# EUROPEAN PATENT APPLICATION

(11) **EP 1 041 147 A2**
(43) Date of publication of application: **04.10.2000**
(21) Application number: 99110507.3
(22) Date of filing: 31.05.1999
(51) Int. Cl.: C12N 15/12, C07K 14/47, C12N 15/79, C12N 5/10, C07K 16/18, A61K 48/00, A61K 38/17

(54) **Microtubule-associated protein TPX2**

(30) Priority: 01.04.1999 EP 99106710
(71) Applicant: EUROPÄISCHES LABORATORIUM FÜR MOLEKULARBIOLOGIE (EMBL), D-69117 Heidelberg (DE)
(72) Inventor: The designation of the inventor has not yet been filed
(74) Representative: Böhm, Brigitte, Dipl.-Chem. Dr.

(57) **Abstract**

A microtubule-associated protein is described which is involved in mechanisms during mitosis.

## Description

The present invention relates to TPX2, a novel microtubule-associated protein which is involved in mechanisms during mitosis.

During mitosis, chromosomes are segregated by a complex microtubule-based structure, the mitotic spindle. The chromatides of each chromosome separate and migrate under the influence of the mitotic spindle to one of the two cell poles each.

Current models of spindle assembly involve the action of a variety of mictrotubule motors exerting forces on microtubules (Barton et al, Proc. Natl. Acad. Sci. USA 93 (1996), 1735-1742; Vernos et al, Curr. Opin. Cell Biol. 8 (1996), 4-9). Kinesin, for example, is a mechano-chemical enzyme acting as microtubule motor. Xklp2 is a plus end-directed Xenopus kinesin-like protein localized at spindle poles and required for centrosome separation during spindle assembly in Xenopus egg extracts (H. Boleti et al., Cell 84 (1996) 49 - 59; E. Karsenti et al., Sem. Dev. Cell. Biol., 7 (1996) 367 - 378).

To understand the functioning of motors in spindle morphogenesis, further investigations are necessary. In particular, different levels of their functions have to be examined. One level concerns the mechanisms of motor localization through specific interactions between the variable nonmotor domains and their cargoes (Afshar et al, Cell 81 (1995), 129-138). A second level relates to the actual motor function in relation to the activity of other plus and minus end-directed motors (Hoyt et al, J. Cell Biol. 118 (1992), 109-120). A third level concerns the regulation of the activity and localization of motors in time and space by posttranslational modifications (Blangy et al, Cell 83 (1995), 1159-1169).

Recently, the localization of the kinesin-like protein Xklp2 to spindle poles was reported as being dependent on the dimerization of Xklp2, a COOH-terminal leucine zipper, a microtubule-associated protein (MAP), and the activity of the dynein-dynactin complex (Wittmann et al, J. Cell Biol. 143 (3) (1998), 673-685). A structure or the sequence of the involved microtubule-associated protein was, however, not revealed.

It was therefore an object of the present invention to provide a novel microtubule-associated protein which is involved in mitosis.

The invention comprises a nucleic acid coding for TPX2 comprising:
(a) the nucleotide sequence shown in SEQ ID NO:1, SEQ ID NO:4 or SEQ ID NO: 6,
(b) a sequence corresponding to the nucleotide sequence shown in SEQ ID NO:1, SEQ ID NO:4 or SEQ ID NO:6 within the degeneration of the genetic code,
(c) a sequence having a homology greater than 80 % to the nucleotide sequence of SEQ ID NO:1, SEQ ID NO:4 or SEQ ID NO:6
(d) a sequence being a section of the nucleotide sequence of (a), (b) or/and (c) having at least 50 bases therefrom,
(e) a sequence which hybridizes with at least one of the sequences (a) to (d) under stringent conditions,
(f) a genomic sequence containing one of the sequences (a) to (e) and further containing one or more introns, or
(g) a sequence which differs from a sequence (a) to (f) due to its origin from a different species.

The term TPX2 is short for "targeting protein for Xklp2". However, not only proteins from Xenopus are comprised by the term TPX2 as used herein but also corresponding proteins from other species, in particular proteins from mammals, preferably humans.

TPX2 functions by assisting the binding of kinesin-like proteins (KLP) having a motor function to microtubules during mitosis. TPX2 itself preferably binds directly to microtubules or/and kinesin-like proteins (KLP), in particular Xklp2 or human kinesin-like protein. Preferably, TPX2 is a receptor for a leucine zipper of the kinesin-like protein. Xklp2 for example was found to have a leucine zipper at the COOH-terminus.

In general TPX2 mediates the binding of kinesin-like proteins to microtubules independent of the species from which it is derived. In particular it mediates the binding of the COOH-terminal domain of kinesin-like proteins, preferably Xklp2, to microtubules. It was found that the protein encoded by SEQ ID NO:1 mediates the binding of Xklp2 as well as the binding of GST(glutathione-S-transferase)-Xklp2-tail to microtubules, indicating that the COOH-terminal domain of KLP is involved in the binding mechanism.

TPX2, however, need not be the only protein required for the localization of the kinesin-like protein on the microtubules, but other prtoteins may be involved as well. An example for such an other protein is the dynein-dynactin complex.

The nucleotide acid according to the invention preferably is a DNA. However, it may also be a cDNA, an RNA or a nucleic acid analog, such as a peptidic nucleic acid or a modified nucleic acid. Such modified nucleic acids may carry for example additional substituents on the nucleobases, the sugar moieties or the phosphate linking groups. Such substituents may alter the properties of the nucleic acid with regard to the desired application.

In one embodiment of the invention, the nucleic acid comprises a sequence having a homology of greater than 80 %, preferably greater than 90 %, and more preferably greater than 95 % to the nucleotide sequences according to SEQ ID NO:1, SEQ ID NO:4 or SEQ ID NO:6 or to sequences corresponding thereto within the degeneration of the genetic code, while still encoding a protein having the described features.

While the nucleic acid according to the invention preferably comprises one of the sequences shown in SEQ ID NO:1, 4 or 6, it may also comprise only a portion therefrom, as long as the properties of the encoded protein are substantially retained. Such a portion comprises a section being at leat 50, preferably at least 100, more prefarably at least 200, and most preferably at least 500 nucleobases of one of the nucleotide sequences (a), (b) or/and (c) described above. A section comprising the coding sequence (CDS) for the protein is particularly preferred.

The term "hybridization under stringent conditions" according to the present invention is used as described by Sambrook et al (Molecular Cloning, A Laboratory Manual, Cold Spring Harbor Laboratory Press (1989), 1.101-1.104). Preferably, a stringent hybridization according to the present invention is given when after washing for an hour with 1 x SSC and 0.1 % SDS at 50°C, preferably at 55°C, more preferably at 62°C, and most preferably at 68°C, and more preferably for one hour with 0.2 x SSC and 0.1 % SDS at 50°C, preferably at 55°C, more preferably at 62°C, and most preferably at 68°C a positive hybridization signal is still observed. A nucleotide sequence which hybridizes under such washing conditions with any of the sequences of (a) to (d), preferably with the nucleotide sequence shown in SEQ ID NO:1, SEQ ID NO:4 or SEQ ID NO:6, is a nucleotide sequence according to the invention.

The nucleic acid preferably contains the sequence as of SEQ ID NO:1 coding for Xenopus laevis eggs TPX2, the sequence as of SEQ ID NO:3 being the cDNA of Xenopus laevis, the sequence as of SEQ ID NO:4 coding for part of the human TPX2 or the sequence as of SEQ ID NO:6 coding for the human TPX2.

SEQ ID NO:4 or sections therefrom can be used by the skilled artisan to obtain the complete cDNA and genomic DNA of human TPX2 by standard protocols known in the art, e.g. as hybridization probes for screening suitable libraries. The complete open reading frame for human TPX2 is shown in SEQ ID NO:6.

The nucleic acids according to the invention can be obtained using known techniques, e.g. using short sections of the nucleotide sequences disclosed herein as hybridization probe or/and primer. They can, however, also be produced by chemical synthesis.

The nucleic acid according to the present invention preferably is an operative association with an expression control sequence. The expression control sequence is preferably active in eukaryotic cells, most preferably in Xenopus or in mammal cells.

The invention further comprises a recombinant vector containing at least one copy of a nucleic acid according to the invention. This may be a prokaryotic or a eukaryotic vector which contains the nucleic acid according to the invention under the control of an expression signal, in particular a promoter, operator, enhancer etc. Examples of prokaryotic vectors are chromosomal vectors such as bacteriophages and extra-chromosomal vectors such as plasmids, circulary plasmid vectors being particularly preferred. Prokaryotic vectors useful according to the present invention are described in, e.g., Sambrook et al, supra. Such prokaryotic vectors can be used to introduce the nucleic acid of interest into a eukaryotic cell.

More preferably, the vector according to the invention is a eukaryotic vector, in particular a vector for mammal cells, preferably a vector for human cells. Most preferred are vectors suitable for gene therapy, such as retrovirus, modified adenovirus or adeno-associated virus. Such vectors are known to the man skilled in the art and are also described, e.g., in Sambrook et al, supra.

The invention further comprises a cell transformed with a nucleic acid or a vector according to the invention. The cell may be a eukaryotic or prokaryotic cell, eukaryotic cells being preferred. The cell may be used as bioreactor to produce the protein of interest.

The invention also comprises a polypeptide encoded by a nucleic acid according to the invention, in particular a polypeptide comprising SEQ ID NO:2, SEQ ID NO:5 or SEQ ID NO:7. It also relates to polypeptides differing therefrom by additions, substitutions, deletions or/and insertions of amino acids that do not substantially affect its activity. The modifications preferably concern single amino acids or short amino acid sections having less than 10, preferably less than 5 amino acids.

The polypeptide is, e.g., obtainable by expression of the corresponding nucleic acid sequence in a suitable expression system (cf. Sambrook et al, supra). The polypeptide can also be fused to other polypeptides or/and proteins to build a fusion protein.

The polypeptide according to the invention, or fragments thereof, can be used for the production of an inhibitor of the respective polypeptide. In particular it can be used as immunogen for the production of antibodies, whereby standard protocols for obtaining antibodies may be used. Since KLP function is essential for mitotic spindle assembly, such inhibitors, preferably antibodies, which interfere with TPX2 are useful to target specifically mitotic cells, and in particular for the treatment of cancer.

Further, the present invention also encompasses a pharmaceutical composition comprising as active component a nucleic acid, vector, polypeptide or inhibitor as described herein. The pharmaceutical composition may additionally comprise pharmaceutically acceptable carriers, vehicles and/or additives and additional active components, if desired. Such a pharmaceutical composition can be used for diagnostic or/and therapeutic purposes. Particularly preferred is the use for affecting mitotic cells and, preferably, for the treatment of cancer.

Since TPX2 is involved in the localization of kinesin-like protein to the microtubules during mitosis, which KLP function in turn is essential for centrosome separation during prophase and the assembly of a bipolar spindle, mitosis can be affected by the addition of TPX2 or its antagonist, respectively. An effect on mitosis can take place on both the nuleic acid and the polypeptide level.

Therefore a use of the nucleic acid according to the invention is in the production of an inhibitor of said nucleic acid. Such an inhibitor can be produced by techniques known to the man skilled in the art. The inhibitor interferes with the activity of the nucleic acid and can be used to inhibit or prevent mitosis of cells, which is of particular interest in cells showing abnormally enhanced mitosis.

On the other hand, the nucleic acid according to the invention can be used directly to induce or/and promote mitosis or to compensate for a cell deficiency.

The active component on nucleic acid level may be formulated into a pharmaceutical composition by techniques known in the art, e.g. using liposomes as carrier or using suitable vectors, e.g. herpes vectors.

More preferably, a polypeptide having TPX2 activity or its antagonist, respectively, are used for affecting mitosis rather than a nucleic acid. Again, a polypeptide having TPX2 activity can be used for inducing and/or promoting mitosis or for the compensation of cell deficiencies.

An inhibitor, preferably an antibody, which interferes with TPX2 activity, can be used to inhibit or/and prevent mitosis, which is particularly useful to target cancer cells.

Since TPX2 is involved in mitosis through the binding of Xklp2-tail to microtubules, this gene and protein, respectively, can also be used to design drug target structures.

The invention is further illustrated by the appending sequences and figures wherein

| | |
|---|---|
| SEQ ID NO:1 | shows the DNA sequence for Xenopus laevis TPX2; |
| SEQ ID NO:2 | shows the Xenopus laevis TPX2 protein sequence (715 amino acids); |
| SEQ ID NO:3 | shows the Xenopus laevis TPX2 cDNA sequence (3539 kb); |
| SEQ ID NO:4 | shows part of the coding sequence for human TPX2; |
| SEQ ID NO:5 | shows the partial protein sequence of human TPX2; |
| SEQ ID NO:6 | shows the open reading frame of the human TPX2 and |
| SEQ ID NO:7 | shows the protein sequence of the human TPX2. |

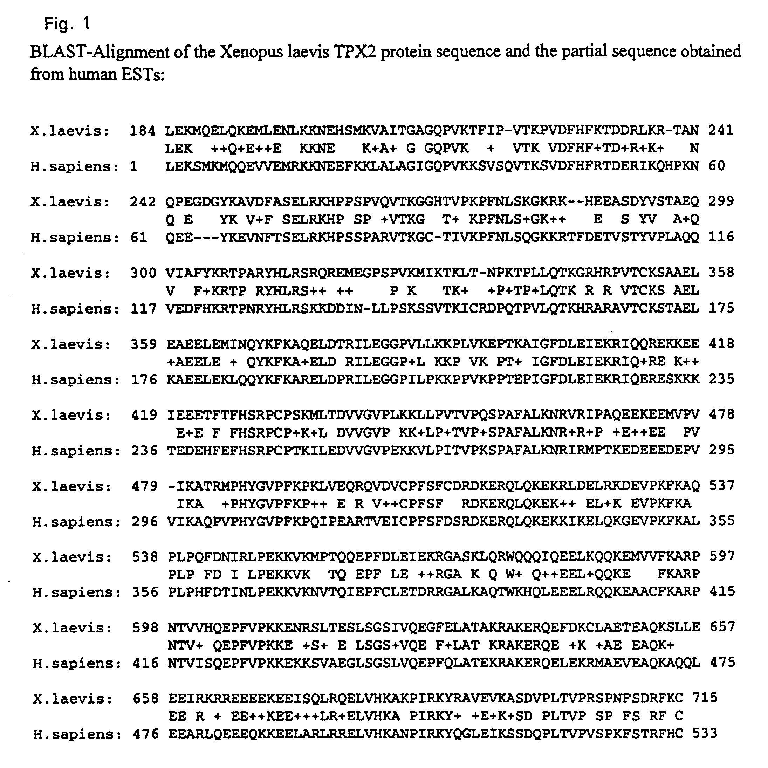

- Figure 1: shows a BLAST-alignment of the Xenopus laevis TPX2 protein sequence and the partial sequence obtained from human ESTs.

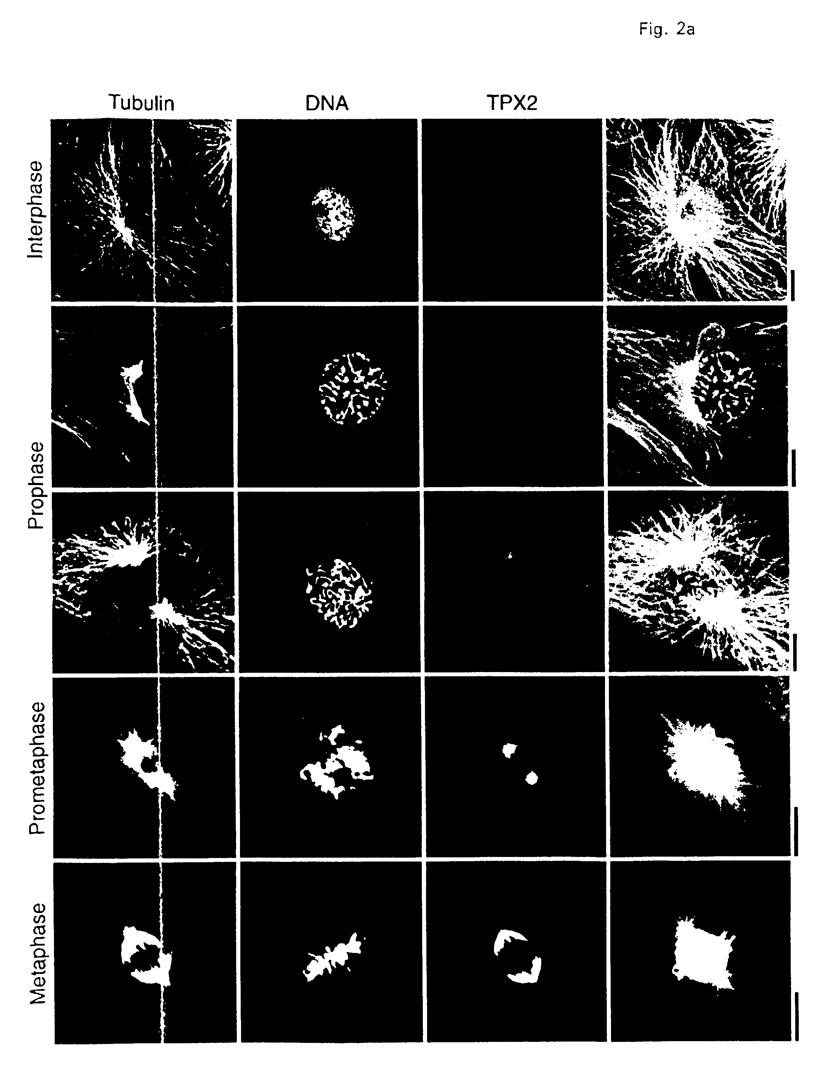

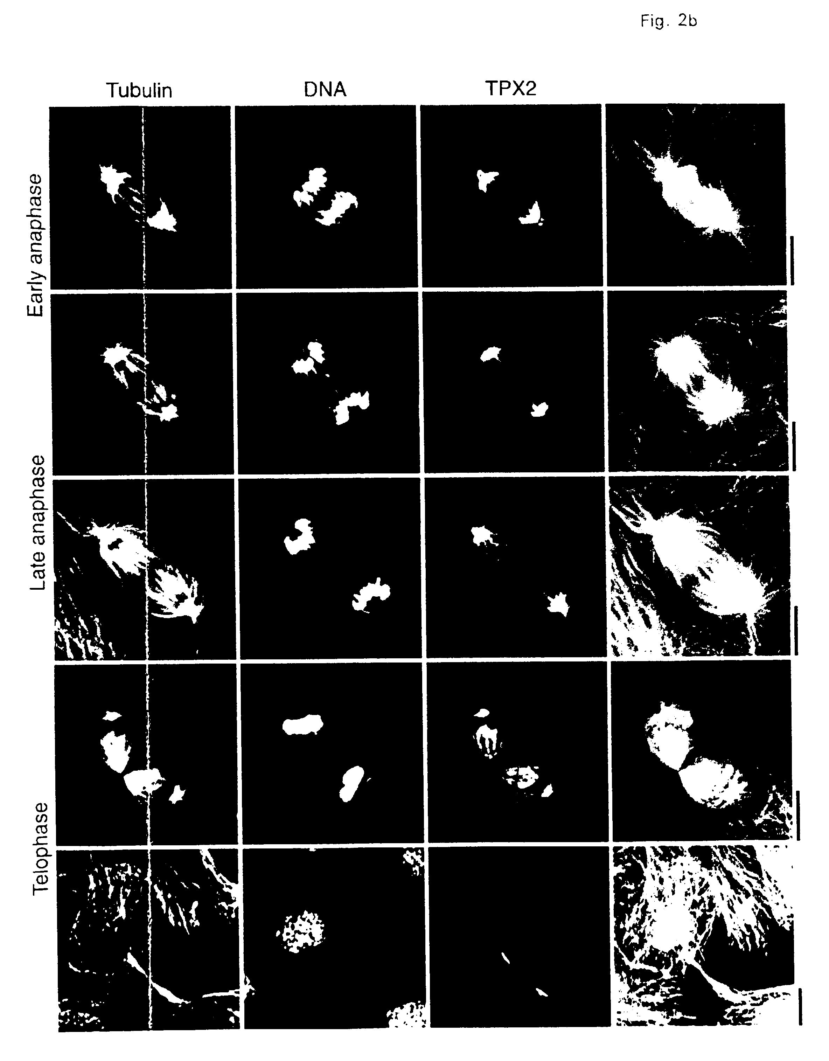

- Figure 2 (two pages):: shows localization of TPX2 throughout the cell cycle. Methanol-fixed XL177 cells were stained with an affinity-purified anti-TPX2 antibody (#5843, 3^{rd} bleed, used at a concentration of 0.6 µg/ml), a monoclonal anti-tubulin antibody and Hoechst No. 33258. FITC-labeled anti-mouse and TRITC-labeled anti-rabbit antibodies were used as secondary antibodies. Images represent single confocal slices. The same staining pattern as with the #5837 serum is observed. TPX2-staining appears at the end of prophase, persists at the spindle poles throughout mitosis until late anaphase. It then relocalizes to the midbody where it disappears in late telophase. Bars, 10 µm.
- Figure 3: shows HeLa cells in metaphase. The cells were fixed in cold methanol and stained with Hoechst No. 33258 and both affinity purified anti-TPX2 antibodies (at a concentration of 2-3 µg/ml). Both antibodies strongly stain the mitotic spindle. However, the cytoplasmic background is higher than in XL177 cells probably due to the higher antibody concentration that was necessary. Bar, 10 µm.
- Figure 4: shows phosphorylation of endogenous TPX2 in mitotic, M, and interphase, I, egg extract incubated in the presence of [γ-³²P]ATP. Taxol was added to 2 µM concentration and the extracts were incubated for 30 min at 20°C. The immunoprecipitates were analyzed on a 6% SDS-PAGE and subjected to autoradiography. TPX2 is phosphorylated in mitotic extract and hyperphosphorylated upon assembly of microtubules.

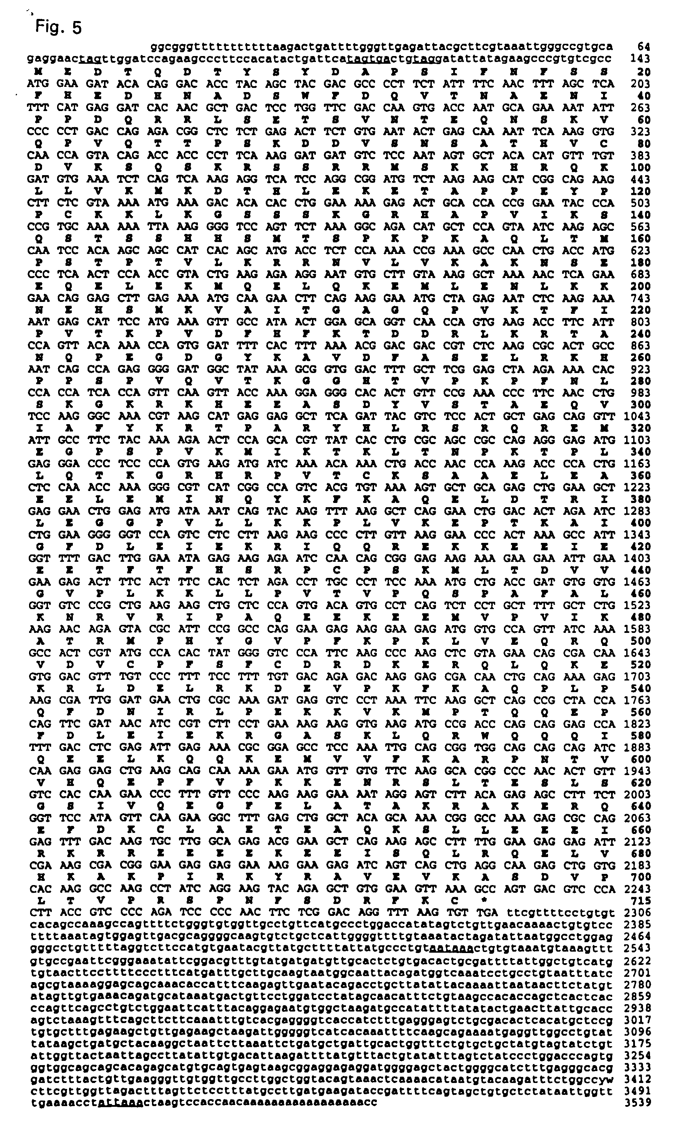

- Figure 5: shows the DNA sequence for Xenopus laevis TPX2 and the Xenopus laevis TPX2 protein sequence.

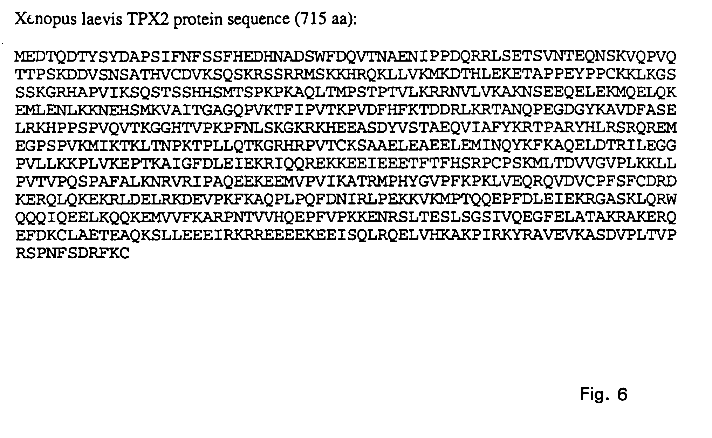

- Figure 6: shows the Xenopus laevis TPX2 protein sequence (715 aa).

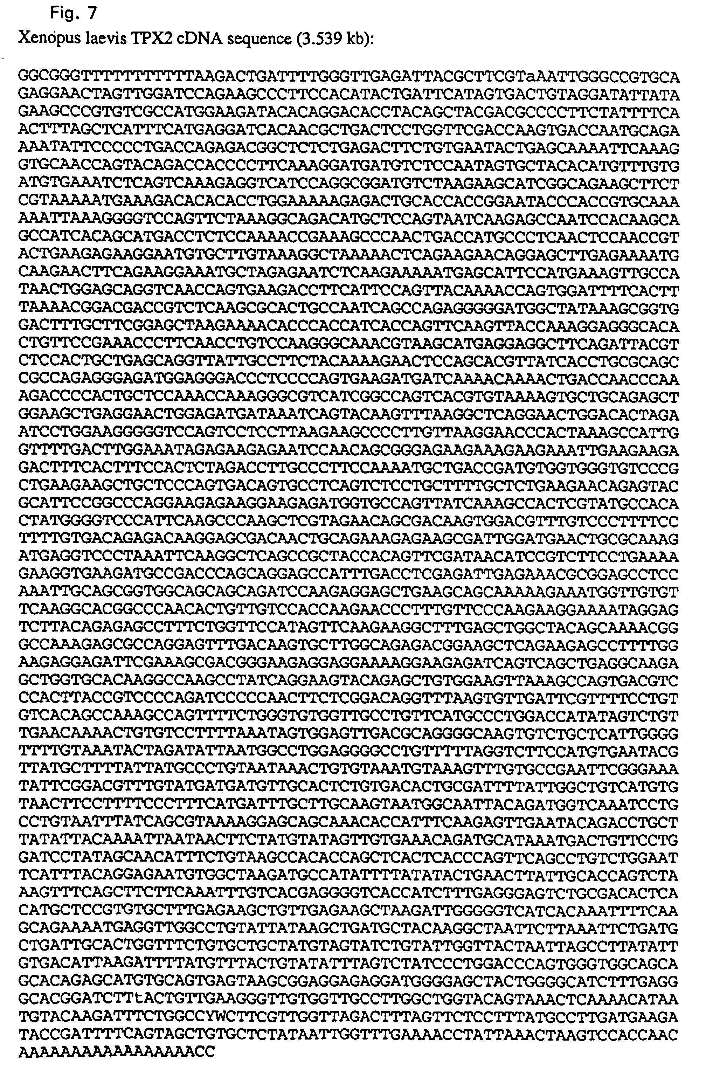

- Figure 7: shows the Xenopus laevis TPX2 cDNA sequence (3.539 kb).

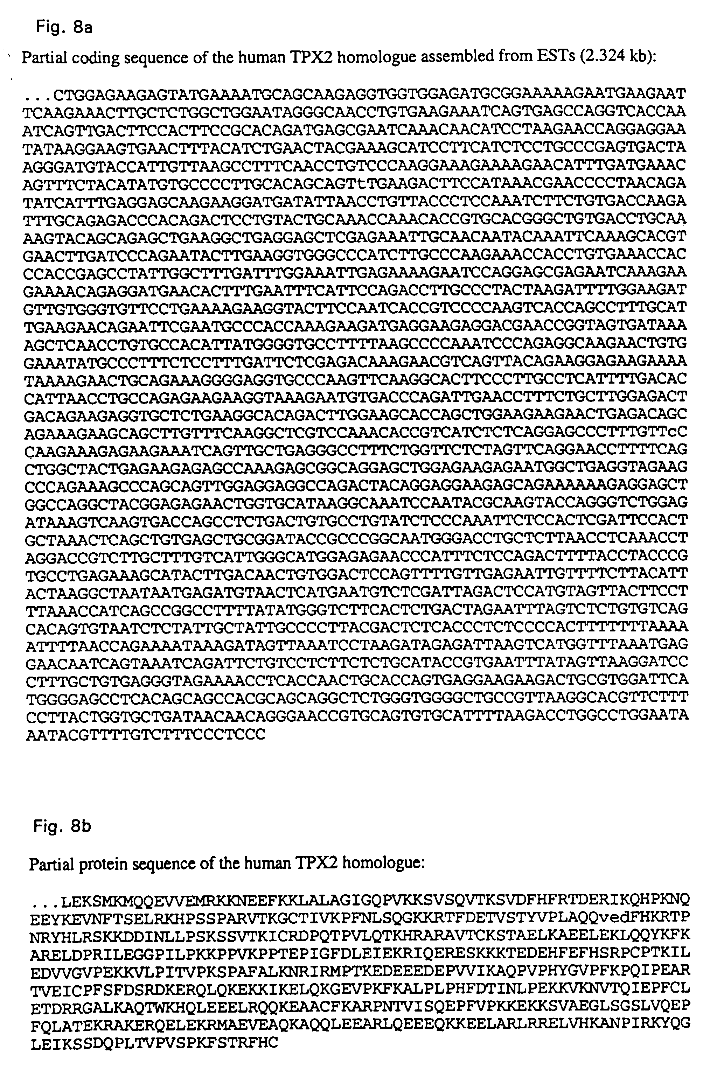

- Figure 8a: shows the partial coding sequence of the human TPX2 homologue assembled from ESTs (2.324 kb).
- Figure 8b: shows the partial protein sequence of the human TPX2 homologue.

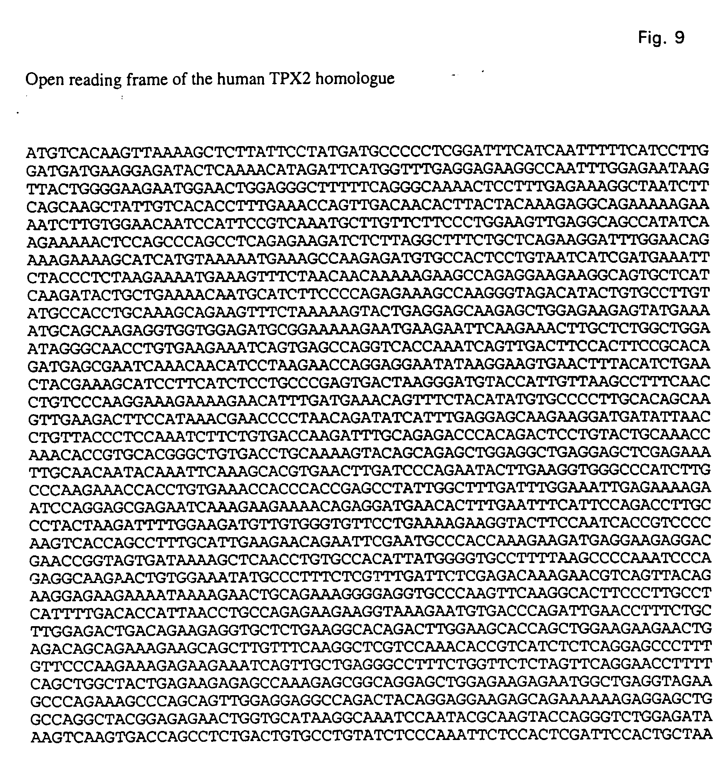

- Figure 9: shows the human TPX2 DNA-sequence.

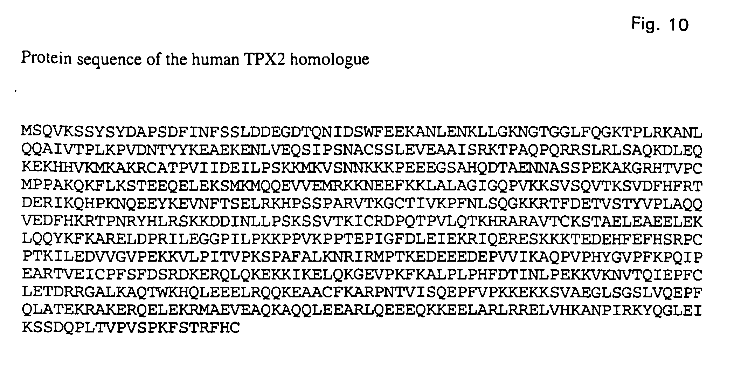

- Figure 10: shows the protein sequence of the human TPX2 homologue.

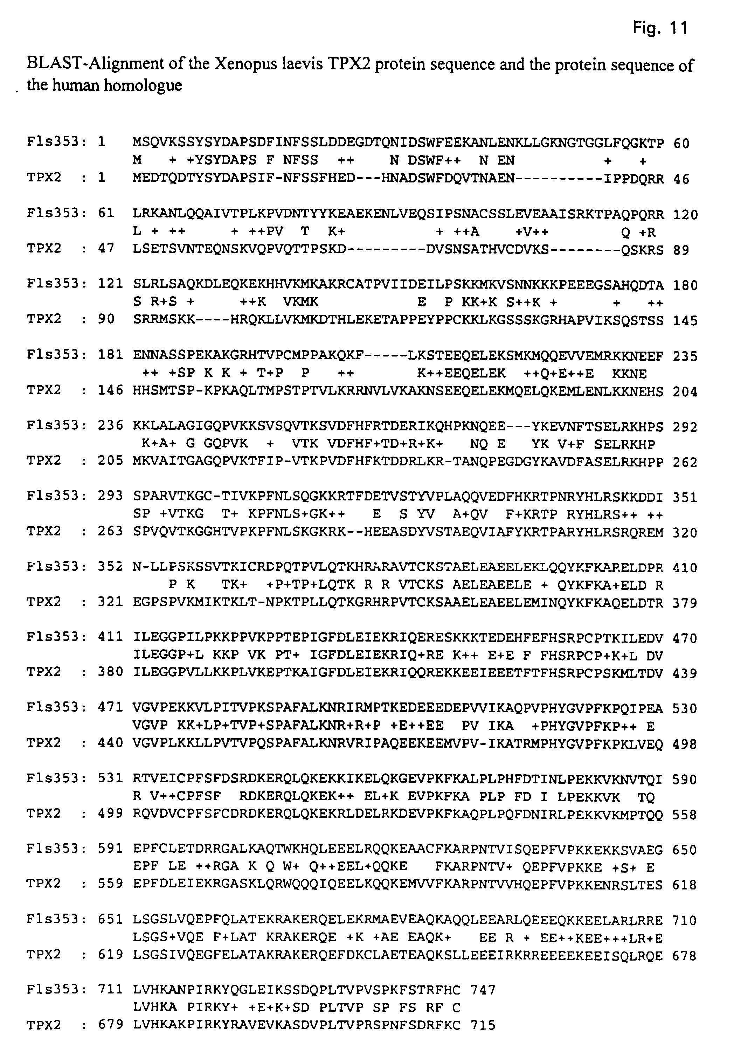

- Figure 11: shows a BLAST-Alignment of the Xenopus laevis TPX2 protein sequence and the protein sequence of the human homologue.

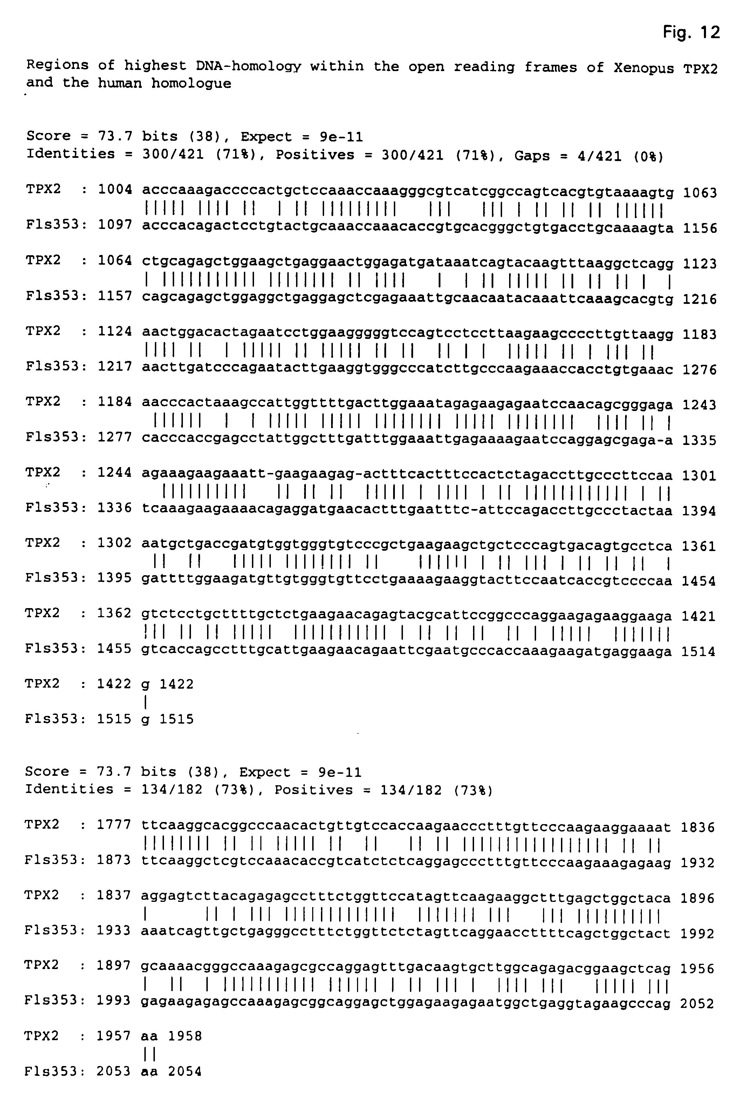

- Figure 12: shows regions of highest DNA-homology within the open reading frames of Xenopus TPX2 and the human homologue.

### Examples

### Example 1: Purification of TPX2

### 1.1 Xenopus egg extracts

CSF-arrested extracts (mitotic extracts) were prepared according to Murray, Cell Cycle Extracts, in Methods in Cell Biology, Vol. 36, B.K. Kay and H.B. Peng, editors, Academic Press, San Diego, CA(1991), 581-605. They were released to interphase by addition of 0.5 mM CaCl₂ and 200 µg/ml cycloheximide and subsequent incubation at 20°C for 45-60 min. High speed extracts were centrifuged for 60 min at 150,000 g at 4°C.

### 1.2 Purification

To determine the additional protein which is required for GST-Xklp2-tail (a glutathione-S-transferase fusion protein containing the COOH-terminal domain of Xklp2 (Boleti et al., Cell 84 (1996) 49-59)) binding to microtubules MAPs were prepared from CSF-arrested egg extract. CSF-arrested egg extract was diluted with two volumes of motor buffer (100 mM K-PIPES, pH 7.0, 0.5 mM EGTA, 2.5 mM magnesium acetate, and 1 mM DTT) containing 10 µg/ml pepstatin, leupeptin, aprotinin, and 1 mM PMSF, and then centrifuged for 90 min at 180,000 g at 4°C. The cytoplasmic layer was collected and supplemented with 0.6 mg/ml bovine brain tubulin (Ashford et al, Preparation of tubulin from bovine brain, in Cell Biology: A Laboratory Handbook, vol. 2, J.E. Celis, editor, Academic Press, San Diego, CA (1998), 205-212) and 20 µM taxol and microtubules were polymerized at room temperature for 30 min. The microtubules were then centrifuged through a 15 % sucrose cushion in motor buffer (30,000 g, 20 min, 22°C ) containing 5 µM taxol. The supernatant was discarded, the cushion once washed with water, removed and the microtubule pellet resuspended in 1/3 of the original volume in motor buffer and taxol, and then centrifuged through a sucrose cushion again. GST-Xklp2-tail alone does not bind to pure taxol-stabilized microtubules. However, a fraction of MAPs contains an activity that mediates the binding of GST-Xklp2-tail to microtubules. Only in the presence of the MAP-fraction a substantial amount of GST-Xklp2-tail was recovered in the microtubule pellet. It was observed by immunofluorescence that in this case GST-Xklp2-tail bound all along the prepolymerized microtubules. These results indicate the presence of a factor in mitotic egg extracts required for the binding of the COOH-terminal domain of Xklp2 microtubules that was enriched in a MAP fraction prepared from CSF-arrested egg extract.

### 1.3 Further purification

As a further purification step, mitotic MAPs were eluted from microtubules in 100 mM steps of NaCI in motor buffer for 15 min at room temperature. Between the elution steps microtubules were recovered by centrifugation (30,000 g, 15 min, 22°C). TPX2 was enriched in the fraction eluted with 300 mM NaCI.

### 1.4 Mono S column

The fraction was diluted to reduce the salt concentration and applied onto a PC 1.6/5 Mono S column (SMART system; Pharmacia Biotech Sverige). The column was eluted with a 1-ml linear gradient of 100-500 mM KCI in 20 mM K-PIPES, pH 7.0, 10 % glycerol, 1 mM EDTA, 1 mM DTT, 0.01 % Tween-20 at 4°C at 25 µl/min and 50-µl fractions were collected. TPX2 eluted at about 350 mM KCI in a single peak, corresponding to a doublet of polypeptide with molecular masses of about 100 kD. The strong affinity of these proteins for the Mono S suggested that they are highly basic.

### 1.5 Superdex 200 gel filtration column

Since the Mono S peak fraction still contained some minor contaminants, it was further purified on a Superdex 200 gel filtration column. The peak fraction from the Mono S chromatography was applied to a PC 3.2/30 Superdex 200 gel filtration column (SMART system; Pharmacia Biotech Sverige) equilibrated with 20 mM K-Hepes, pH 7.0, 300 mM KCI, 10 % glycerol, 1 mM EDTA, 1 mM DTT, and 0.01 % Tween-20 at 4°C. The column was eluted with the same buffer at a flow rate of 20 µ/min and 40-µl fractions were collected. The column fractions were assayed in the following way: 4 mg/ml cycled bovine brain tubulin was polymerized in BRB80 containing 5 mM MgCl₂, 33 % glycerol and 1 mM GTP at 37°C for 30 min. The microtubules were stabilized by the addition of 20 µM taxol. 5 µl MAPs or column fractions were mixed with 10 µl BRB80 containing 1 mM DTT, 5 µM taxol, 0.1 % Triton X-100 and 1 µM GST-Xklp2-tail. 5 µl of the prepolymerized microtubules were added and the reactions incubated at 20°C for 30 min. The reactions were then diluted 1:5 with BRB80 containing 1 mM DTT, 5 µM taxol, and 0.1 % Triton X-100, and then centrifuged through a 10 % sucrose cushion in BRB80, 5 µM taxol at 200,000 g at 20°C for 15 min. The cushion was washed once with water and the microtubule pellet solubilized in SDS-PAGE sample buffer and analyzed by Western blotting with an anti-GST antibody.

The anti-GST antibody was affinity purified against GST (glutathion-S-transferase) from a rabbit serum immunized with an unrelated GST-fusion protein.

Obtained was a MAP which is named TPX2 herein (targeting protein for Xklp2) and mediates the binding of the COOH-terminal domain of Xklp2 to microtubules. Purifed TPX2 itself is capable of rebinding to pure microtubules. There is a high probability that TPX2 is the receptor for the leucine zipper found at the COOH-terminus of Xklp2.

### Example 2: Cloning of TPX2

Sequencing of the purified protein TPX2 obtained in Example 1 was performed. Subsequently, a polymerase chain reaction (PCR) with degenerate oligos was conducted, followed by screening of a Xenopus cDNA library.

### 2.1 Obtaining TPX2 peptide sequence

TPX2 was purified from mitotic egg extract as described in Example 1. The peak fractions after Mono S chromatography of several preparations from about 60-70 ml of crude extract altogether were pooled and run on a preparative protein gel. After Coomassie-staining, the 100-kD band corresponding to TPX2 was cut out and the protein digested with trypsin. The tryptic peptides were then subjected to peptide sequencing by tandem electrospray mass spectroscopy.

Several peptide sequences were obtained by mass spectroscopy that proved to be useful for designing primers (Table 1). Mass spectroscopic sequencing can not distinguish between leucine and isoleucine because of their identical mass leading to an ambiguity in these positions. Furthermore, for two peptides two slightly divergent sequences were obtained either reflecting sequencing errors or different isoforms present in the purified TPX2 fraction. Database searches with these peptide sequences did not reveal any similarities to known proteins.

**Table 1**

| | |
|---|---|
| Table 1: TPX2 peptide sequences obtained by tandem electrospray mass spectroscopy and the derived degenerate oligonucleotides used for PCR. The peptide sequences indicated in bold were used for primer design. Brackets indicate uncertainties. (t) These peptide sequences were confirmed by Edman degradation and, therefore, are not ambigous at the leucine positions. Standard degenerate nucleotid alphabet: R(A/G), Y(C/T), K(G/T), M(A/C), S(G/C), W(A/T), B(G/C/T), D(A/G/T), H(A/C/T), V(A/C/G), N(any), and l(inosine). | |

| **Peptide sequence** | **Degenerate oligonucleotides** |
|---|---|
| ...(V)TVPQSPAFA(L/I)K | |
| (L/I)(L/I)**PVTVPQSPA**FPSK | A5: CCI GTN ACI GTN CCI CAR WSI CCN GC |
| | A3: GC NGG ISW YTG IGG IAC NGT IAC NGG |
| **...GFD(L/I)E(L/I)EQ**R | B5: GGI TTY GAY HTI GAR HTN GAR C |
| | B3: TG YTC IAD YTC IAD RTC RAA NCC |
| ILEGGPVLLK^{†} | |
| I**LEGGPVLPK**^{†} | C5: TN GAR GGI GGN CCI GTI YTN CC |
| | C3: YTT IGG NAR IAC NGG ICC NCC YTC |
| **AVDFASE**IR^{†} | D5: GCN GTI GAY TTY GCI WSN GAR |
| | D3: YTC ISW NGC RAA RTC IAC NGC |
| (TQ)**PVDFGVQ**K | E5: CCN GTI GAY TTY GGN GTN C |
| | E3: TG NAC ICC RAA RTC NAC NGG |
| (L/I)(L/I)EYF(L/I)(L/I)K | |

### 2.2 RNA isolation, cDNA synthesis and degenerate PCR

Since the order of the peptides in the TPX2 protein sequence was unknown, five pairs of degenerate oligonucleotides were designed pointing in both the upstream and downstream direction (Table 1) based on the least ambiguous parts of the peptide sequences. The primers were 19-26 nucleotides long and contained up to four inosines to keep the degeneracy low (256-576 fold) taking into account all possible permutations of the amino acid sequence. Inosine presumably base pairs equally well with all four nucleotides.

Poly(A)⁺-mRNA was isolated from approximately 1 mg of total *Xenopus* egg RNA using the PolyATract mRNA Isolation System (Promega) following the suppliers instructions. Briefly, the RNA is hybridized to a biotinylated oligo(dT) primer that is captured and washed at high stringency using streptavidin coupled paramagnetic beads. The poly(A)⁺-mRNA was precipitated in the presence of 625 mM ammonium acetate, 1 µg glycogen and 66% ethanol at -70°C for 30 min.
After centrifugation and drying, the RNA pellet was dissolved in sterile water containing 25 ng oligo(dT)₂₂ heated to 70°C for 10 min and chilled on ice. The reaction was then adjusted to 50 µl 50 mM Tris-HCl pH 8.3, 75 mM KCl, 3 mM MgCl₂, 10 mM DTT, 1 unit RNAse block (Stratagene) and 0.5 mM dNTPs and prewarmed to 42°C. 500 units Superscript II reverse transcriptase (Gibco BRL) were added and cDNA was synthesized at 42°C for 90 min. The reaction was terminated by incubation at 70°C for 15 min. This mixture was directly used as a template for PCR.

The degenerate PCR was performed in 50 pI reactions containing 20 mM Tris-HCI pH 8.4, 50 mM KCl, 1.5 mM MgCl₂, 300 µM dNTPs, 5 units AmpliTaq (Perkin-Elmer), 1 µM of the degenerate primers in all possible combinations and 0.1 to 0.5 µl of the RNA/cDNA template using the following program:

| | | |
|---|---|---|
| 94°C | 2 min | |
| 40°C or 50°C | 1 min | |
| 72°C | 1 min | |
| 94°C | 30 sec | \| |
| 40°C or 50°C | 30 sec | \| 30 - 35 cycles |
| 72°C | 1 min | \| |
| 72°C | 5 min | |

After polishing the ends with Pfu DNA polymerase, the PCR-fragments were subcloned with the pCR-Script Amp SK(+) cloning kit (Stratagene).

### 2.3 Library screening

| | | |
|---|---|---|
| 20x SSC, pH 7.0: | 3 M | NaCl |
| | 0.3 M | Trisodium citrate |

Library screening was done according to standard protocols (Ausubel et al., 1995. Short Protocols in Molecular Biology. John Wiley & Sons, Inc.). The PCR-fragments obtained with the primer pairs D5/C3 and C5/A3 were radiolabeled by random priming and DNA synthesis in the presence of [α³²P]dCTP (Amersham LifeScience) using High Prime (Boehringer Mannheim), purified on Sephadex G-50 gel filtration columns (NICK column from Pharmacia Biotech) and used to screen a mature *Xenopus* oocyte cDNA library in λ-ZAP (obtained from John Shuttleworth, University of Birmingham). The library contained the inserts in the EcoRI site of the pBluescript phagemid.

Phages were grown in top agar containing a lawn of *Escherichia coli* BB4 cells. Plaque lifts were taken with Hybond-N nylon filters (Amersham Life Science) for 30-60 seconds, denatured in 0.5 M NaOH, 1.5 M NaCI for 1 min, neutralized in 0.5 M Tris-HCI pH 8.0, 1.5 M NaCI for 5 min and finally accumulated in 2x SSC. The DNA was cross-linked to the filters using an UV Stratalinker 2400 (Stratagene).

The filters were prehybridized in 50% deionized formamide, 1 M NaCl, 1% SDS, 10% dextran sulphate and 100 µg/ml sheared denatured salmon sperm DNA for 2-3 hours at 42°C. Subsequently, the radiolabeled probe was added and hybridized at 42°C overnight. The filters were washed twice in 2x SSC, 0.5% SDS, twice in 1x SSC, 0.5% SDS and once in 0.1x SSC, 0.1% SDS at 65°C for 15 min each.

Positive plaques were cut out and eluted with approximately 0.5 ml 50 mM Tris-HCI pH 7.5, 100 mM NaCI, 8 mM MgSO₄, 0.01 % gelatin containing a drop of chloroform for 1-2 hours at 4°C, diluted and plated for the next round of screening.

After three rounds of screening, positive pBluescript phagemids were isolated by in vivo excision using the ExAssist/SOLR system (Stratagene) following the suppliers instructions.
To fully sequence TPX2 clone #8 that produced the strongest band in the an vitro transcripiton/translation reaction was selected and progressive unidirectional deletions were prepared by digestion with exonuclease III using the Erase-a-Base System (Promega). The plasmid was either cut with Kpnl and Sal I or with Sacl and Notl to generate a nuclease-resistant and a nuclease-sensitive end close to the T7 or the T3 promoter, respectively. The exonuclease III reaction was done according to the suppliers instructions taking timepoints every 30-40 seconds. Sequencing was done with standard T7 or T3 primers.

Clone #8 contained one long open reading frame of 2145 bp starting at position 203. Probably the first ATG in the sequence also represented the start codon of the open reading frame since it was preceded by several in frame stop codons and the surrounding nucleotides matched reasonably well the consensus for eukaryotic translation initiation (GCC(A/G)CCATGG).

### 2.4 Analysis of the TPX2 predicted amino acid sequence

The open reading frame of clone #8 encoded a polypeptide of 715 amino acids with a predicted size of 82.4 kD. The predicted protein is highly charged (18.7 % strongly basic and 14.7 % strongly acidic amino acids) and extraordinarily basic (the predicted isoelectric point is about 9.5) possibly explaining its strong interaction with the cation exchanger resin during purification.

Apart from the last peptide shown in Table 1 that was not very specific in the first place, all other peptides obtained by mass spectroscopy could be identified in the predicted protein sequence. Minor differences may be due to peptide sequencing errors or the existence of different isoforms of the protein. Most of the differences observed between clone #8 and the peptide sequences can be explained by single nucleotide changes.

**Table 2:**

| | | | | |
|---|---|---|---|---|
| Homology of human ESTs to the TPX2 protein sequence. The five most interesting out of 24 significant BLAST hits are listed (from December 1998). The query sequence was filtered for regions of low complexity. | | | | |

| **Accession number** | **Homology in TPX2** | **Identities** | **Similarities** | **Expect** |
|---|---|---|---|---|
| AA828703 | amino acid 424-547 | 60% | 72% | 7e⁻³⁶ |
| AA218615 | amino acid 424-517 | 62% | 70% | 9e⁻³⁵ |
| AA134490 | amino acid 199-317 | 59% | 73% | 5e⁻²⁸ |
| AA159064 | amino acid 226-348 | 56% | 72% | 4e⁻²⁰ |
| AA136254 | amino acid 594-686 | 53% | 60% | 2e⁻¹⁸ |

Database searches with either the nucleotide or the predicted protein sequence did not reveal any obvious similarity to any protein characterized so far. Since TPX2 presumably is a microtubule-binding protein it was specifically compared to other MAPs. It showed no significant homology to NuMA and it was not homologous to the better defined microtubule binding domains of the the tau-family shared by tau, MAP2, MAP4 and chTOG, the human homolog of XMAP215 (Charrasse et al., 1998. The TOGp protein is a new human microtubule-associated protein homologous to the *Xenopus* XMAP215. *J. Cell Sci.* 111:1371-1383.; Drewes et al., 1998. MAPs, MARKs and microtubule dynamics. *Trends Biochem. Sci.* 23:307-311.) or of the CLIP-170/p150^{Glued} type (Pierre et al., 1992. CLIP-170 links endocytic vesicles to microtubules. *Cell.* 70:887-900.; Waterman-Storer et al., 1995. The p150^{Glued} component of the dynactin complex binds to both microtubules and the actin-related protein centractin (Arp-1). *Proc. Natl. Acad. Sci. USA.* 92:1634-1638.).

However, a BLAST search of the EST database revealed a number of vertebrate (mainly mammalian) sequences that showed a very high homology to clone #8 (Table 2). No matches were found to bacterial, yeast or *Caenorhabditis elegans* sequences. Thus, clone #8 encoded a novel protein representing *Xenopus* TPX2.

The TPX2 protein was predicted to be mainly α-helical, but no obvious structural domains were identified apart from two short regions (amino acid 171-208 and 650-684) that showed a significant probability for coiled-coil formation.

A closer look revealed that TPX2 contained seven potential nuclear localization sites conforming either to a 4-residue pattern composed of four basic amino acids or of three basic amino acids and either a histidine or a proline, or to a 7-residue pattern starting with a proline and followed within 3 residues by a basic segment containing 3 basic residues out of 4. However, these nuclear localization sites may be coincidental considering the high density of basic residues in this protein. TPX2 did not contain a bipartite nuclear localization signal.

More interestingly, the TPX2 protein sequence also contained several potential phosphorylation sites. Three consensus phosphorylation sites for protein kinase A, (K/R)(K/R)XS, three sites for cdc2 kinase, (S/T)PX(K/R), and five potential MAP-kinase sites, PX(S/T)P, were identified. The TPX2 protein did not contain a MARK kinase site, KXGS, that has been identified in the microtubule-binding domain of tau-like proteins.

### Example 3: Human TPX homologue

To compare the *Xenopus* TPX2 to the homologous protein of a different species, clones for three of the human ESTs were obtained from the IMAGE consortium and sequenced from both ends. It turned out that all three ESTs originated from the same gene starting at different positions but terminating at the same position, probably the poly(A)-tail. The EST-sequences could be assembled into a putative human TPX2 homologous sequence missing only about 180 amino acids at its NH₂-terminus. A BLAST alignment revealed that the proteins were 63% identical (77% similarity) in this region. Also the human protein has a very basic isoelectric point (about 9.4) and contains a predicted coiled-coil domain at the same position. Both proteins end with a peculiar cysteine as last amino acid. Interestingly, one of the cdc2 sites and all four MAP-kinase sites present in this part of the protein are conserved between frog and human. This suggests that TPX2 function might be regulated by phosphorylation. The nuclear localization sites are less well conserved. The human homologue contains only two putative nuclear localization sites in this region and only one is conserved between frog and human.

### Example 4: Features and properties of TPX2

The protein obtained in Example 2 binds to microtubules and to Xklp2 leucine zipper at the C-terminal domain. During mitosis, the protein TPX2 is localized at spindle poles. It is involved in the localization of Xklp2, a plus end-directed kinesin-like protein, to microtubule minus ends during mitosis. Xklp2 function is essential for centrosome separation during prophase and assembly of a bipolar mitotic spindle. Since Xklp2 function is essential for mitotic spindle assembly, TPX2 is useful for finding drugs interfering therewith by impairing the binding of TPX2 to microtubule minus ends or to Xklp2. Such drugs are useful to target specifically mitotic cells, in particular cancer cells.

### 4.1 Subcellular localization of TPX2

Polyclonal antibodies were raised against a full length GST-TPX2 fusion protein (rabbit #5837) and against a truncated GST-fusion protein (rabbit #5843). Both sera were affinity purified on a column of untagged TPX2 covalently coupled to sepharose. On Western blots both antibodies recognized the recombinant untagged TPX2 as well as a single band of the same size in *Xenopus* egg extracts and in an SDS-lysate of XL177 *Xenopus* tissue culture cells. Interestingly, in mitotic egg extract and in a preparation of mitotic MAPs the band was upshifted fitting better to the apparent molecular weight of TPX2 that was observed in the original purification from mitotic egg extract. The upshift suggested a posttranslational modification, possibly phosphorylation, of TPX2 in mitosis.

Both antibodies were used for immunofluorescence. XL177 cells were grown on coverslips in 70 % Leibovitz L-15 (Sigma Chemical Co.) containing 10 % FCS. The coverslips were briefly rinsed with 70% PBS and fixed in methanol at -20°C for 5-10 min and unspecific binding was blocked by incubation in PBS containing 2% BSA, 0.1 % Triton X-100 for 10 min. Primary and secondary antibodies were diluted in the same buffer and added to the coverslips for 20-30 min. The coverslips were washed 3-4 times with IF buffer after each antibody incubation and at the end embedded in mowiol.

The affinity-purified antibodies yielded a cleaner signal, but also the crude rabbit sera worked well and showed the same staining pattern. XL177 cells grown on coverslips simply fixed in cold methanol without preextraction gave the best results. The staining pattern of glutaraldehyde fixed cells looked similar, but the cytoplasmic background was drastically increased. In interphase cells no prominent staining was observed. In particular, there was no labeling of microtubules or centrosomes. In some cells, the nuclei appeared to be stained above background, but this was not very clear. Also in prophase, when the chromatin started to condense, no particular staining pattern was observed. At some point, possibly around the time of nuclear envelope breakdown, TPX2 staining became apparent at the center of the asters and in prometaphase a bright staining was evident at the center of both asters. The labeling intensity still increased and at metaphase the spindle microtubules were brightly stained. The signal appeared to be more intense towards the spindle poles. Interestingly, astral microtubules were basically not stained. During anaphase, the staining at the spindle poles remained and in early anaphase no labeling was visible in the center of the spindle between the separating sister chromatids. Later, a relocalization to the midzone occured that resulted in a fairly strong staining of the midbody, the cones of overlapping microtubules that remain between the two dividing cells, in late telophase at the end of mitosis, leaving no staining behind in the centrosomal area (Figure 2).

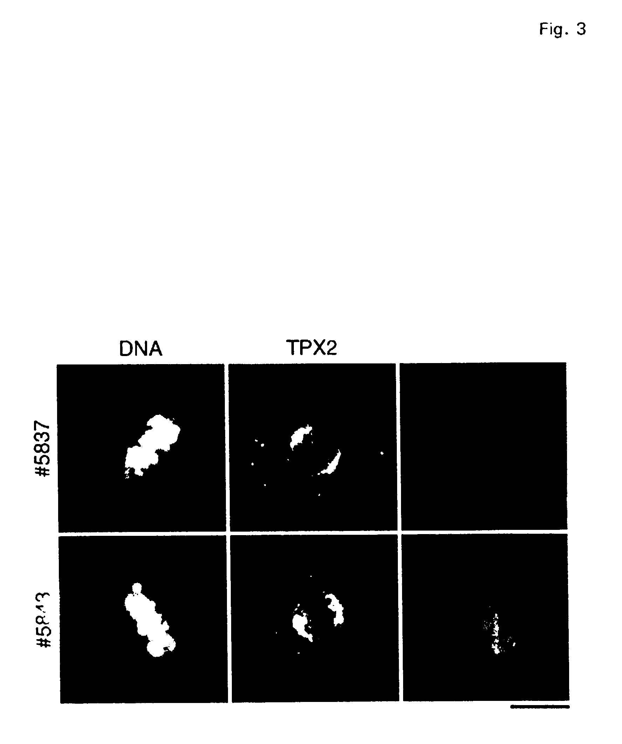

Sequencing of the human ESTs revealed a high degree of homology to TPX2. Therefore, it was also attempted to stain HeLa cells with the two anti-TPX2 antibodies. The antibody concentration had to be raised and the signal to noise ratio was drastically decreased, but it was still possible to observe a staining pattern similar to the one in *Xenopus* cells from prometaphase to anaphase. Especially the mitotic spindle in metaphase was heavily stained (Figure 3).

### 4.2 Phosphorylation in egg extract

| | | |
|---|---|---|
| Stop Buffer, pH 7.5: | 100 mM | NaF |
| | 80 mM | β-glycerophosphate |
| | 20 mM | sodium pyrophosphate, Na₄P₂O₇ |
| | 20 mM | EDTA |
| | 10 µg/ml | aprotinin, pepstatin, leupeptin |
| | 2 µM | microcystin |

AffiPrep protein A beads (BioRad) were washed 2-3 times with PBS containing 0.1 % Triton X-100 and 5-10 µg affinity-purified polyclonal antibody was bound to 10 µl packed beads overnight at 4°C. The next morning, the beads were washed twice with PBS containing 0.1 % Triton X-100 and twice with Stop buffer and were kept resuspended in Stop buffer.

30-50 µCi [γ-³²P]ATP (Amersham LifeScience) were added to 50 µl mitotic or interphase *Xenopus* egg extract and incubated for 20-30 min at 20°C. The reaction was stopped by addition of 100-200 µl Stop buffer containing 10 µl of the antibody beads. The mixture was incubated for one hour on a rotating wheel at 4°C. The beads were washed twice with Stop buffer and twice with PBS containing 0.1 % Triton X-100. At the end, the beads were resuspended in SDS-PAGE sample buffer and analyzed by SDS-PAGE and autoradiography.

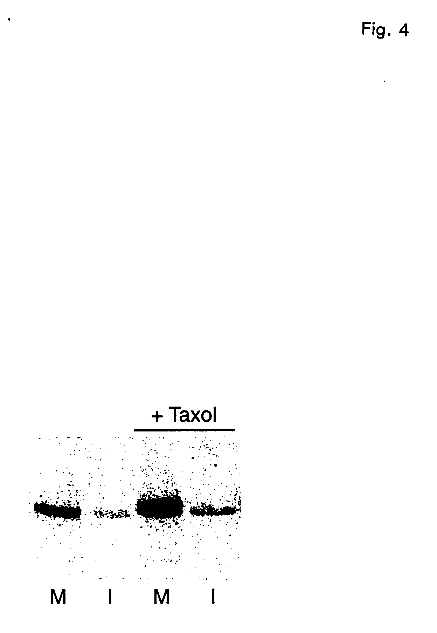

The protein immunoprecipitated from mitotic extract showed a retardation in gel electrophoresis and an increased incorporation of radioactivity indicating that it was specifically phosphorylated in mitosis (Figure 4). More interestingly, a second phosphorylation event was observed in mitotic extract when taxol was added to induce microtubule polymerization. This hyperphosphorylation only occurred in mitotic but not in interphase extract. When the gel was quantified with a phosphoimager the increase in incorporation of radioactivity was about 3-5-fold from interphase to mitotic extract and another 2-3-fold upon addition of taxol to mitotic extract.

## Claims

1. A nucleic acid coding for TPX2 comprising:
(a) the nucleotide sequence shown in SEQ ID NO:1, SEQ ID NO:4 or SEQ ID NO:6
(b) a sequence corresponding to the nucleotide sequence shown in SEQ ID NO:1, SEQ ID NO:4 or SEQ ID NO:6 within the degeneration of the genetic code,
(c) a sequence having a homology greater than 80 % to the nucleotide sequence of SEQ ID NO:1, SEQ ID NO:4 or SEQ ID NO:6
(d) a sequence being a section of the nucleotide sequence of (a), (b) or/and (c) having at least 50 bases therefrom,
(e) a sequence which hybridizes with at least one of the sequences (a) to (d) under stringent conditions,
(f) a genomic sequence containing one of the sequences (a) to (e) and further containing one ore more introns, or
(g) a sequence which differs from a sequence (a) to (f) due to its origin from a different species.

2. A nucleic acid according to claim 1, encoding a protein which is involved in mitosis.

3. A nucleic acid according to claim 1 or 2, encoding a protein which binds to microtubules.

4. A nucleic acid according to any of claims 1 to 3, encoding a protein which binds to kinesin-like proteins.

5. A nucleic acid according to any of claims 1 to 4, characterized in that it contains a cDNA according to SEQ ID NO:3.

6. A recombinant vector characterized in that it contains at least one copy of a nucleic acid according to claims 1 to 5.

7. A vector according to claim 6, characterized in that it is a eukaryotic vector.

8. A cell characterized in that it is transformed with a nucleic acid according to any of claims 1 to 5 or with a vector according to claim 6 or 7.

9. Use of a nucleic acid according to any of claims 1 to 5 for the production of an inhibitor of said nucleic acid.

10. An inhibitor of a nucleic acid according to any of claims 1 to 5.

11. A polypeptide encoded by a nucleic acid according to any of claims 1 to 5.

12. A polypeptide according to claim 11, comprising the sequence shown in SEQ ID NO:2.

13. A polypeptide according to claim 11, comprising the sequence shown in SEQ ID NO:5 or SEQ ID NO:7.

14. A polypeptide according to any of claims 11 to 13, characterized in that it binds to microtubules and/or kinesin-like protein.

15. A polypeptide according to any of claims 11 to 14, characterized in that it contains deletions, substitutions, insertions or/and additions of amino acids that do not substantially affect its activity.

16. A polypeptide according to any of claims 11 to 15, wherein a second protein is fused to build a fusion protein.

17. Use of a polypeptide according to any of claims 11 to 16, or of a fragment thereof for the production of a TPX2-inhibitor.

18. Use according to claim 17 as immunogen for the production of an antibody.

19. An inhibitor of a polypeptide according to any of claims 11 to 16.

20. An inhibitor according to claim 19, characterized in that it is an antibody against the polypeptide.

21. Use of a polypeptide according to any of claims 11 to 16 or of an inhibitor according to claim 17 or 18 for inducing or inhibiting mitosis.

22. A pharmaceutical composition comprising:
(a) a nucleic acid according to any of claims 1 to 5,
(b) a recombinant vector according to claim 6 or 7,
(c) an inhibitor according to claim 10,
(e) a polypeptide according to any of claims 11 to 16, or/and
(f) an inhibitor according to claim 17 or 18.

23. Use of a pharmaceutical composition according to claim 22 for affecting mitotic cells.

24. Use of an inhibitor according to claim 10 or 17 to 18 for the treatment of cancer.


SEQUENCE LISTING

<110> Europaeisches Laboratorium fuer Molerkularbiologie

<120> TPX2

<130> 19742P EP

<140>
<141>

<160> 5

<170> PatentIn Ver. 2.1

<210> 1
<211> 3539
<212> DNA
<213> Xenopus laevis

<220>
<221> CDS
<222> (144)..(2291)

<400> 1
ggcgggtttt tttttttaag actgattttg ggttgagatt acgcttcgta aattgggccg 60

tgcagaggaa ctagttggat ccagaagccc ttccacatac tgattcatag tgactgtagg 120

atattataga agcccgtgtc gcc atg gaa gat aca cag gac acc tac agc tac 173
Met Glu Asp Thr Gln Asp Thr Tyr Ser Tyr
1               5                   10

gac gcc cct tct att ttc aac ttt agc tca ttt cat gag gat cac aac 221
Asp Ala Pro Ser Ile Phe Asn Phe Ser Ser Phe His Glu Asp His Asn
                15                  20                  25

gct gac tcc tgg ttc gac caa gtg acc aat gca gaa aat att ccc cct 269
Ala Asp Ser Trp Phe Asp Gln Val Thr Asn Ala Glu Asn Ile Pro Pro
            30                  35                  40

gac cag aga cgg ctc tct gag act tct gtg aat act gag caa aat tca   317

Asp Gln Arg Arg Leu Ser Glu Thr Ser Val Asn Thr Glu Gln Asn Ser
        45                  50                  55

aag gtg caa cca gta cag acc acc cct tca aag gat gat gtc tcc aat   365

Lys Val Gln Pro Val Gln Thr Thr Pro Ser Lys Asp Asp Val Ser Asn
    60                  65                  70

agt gct aca cat gtt tgt gat gtg aaa tct cag tca aag agg tca tcc   413

Ser Ala Thr His Val Cys Asp Val Lys Ser Gln Ser Lys Arg Ser Ser
75                  80                  85                  90

agg cgg atg tct aag aag cat cgg cag aag ctt ctc gta aaa atg aaa   461

Arg Arg Met Ser Lys Lys His Arg Gln Lys Leu Leu Val Lys Met Lys
                95                  100                 105

gac aca cac ctg gaa aaa gag act gca cca ccg gaa tac cca ccg tgc   509

Asp Thr His Leu Glu Lys Glu Thr Ala Pro Pro Glu Tyr Pro Pro Cys
            110                 115                 120

aaa aaa tta aag ggg tcc agt tct aaa ggc aga cat gct cca gta atc   557

Lys Lys Leu Lys Gly Ser Ser Ser Lys Gly Arg His Ala Pro Val Ile
        125                 130                 135

aag agc caa tcc aca agc agc cat cac agc atg acc tct cca aaa ccg   605

Lys Ser Gln Ser Thr Ser Ser His His Ser Met Thr Ser Pro Lys Pro
    140                 145                 150

aaa gcc caa ctg acc atg ccc tca act cca acc gta ctg aag aga agg   653

Lys Ala Gln Leu Thr Met Pro Ser Thr Pro Thr Val Leu Lys Arg Arg
155                 160                 165                 170

aat gtg ctt gta aag gct aaa aac tca gaa gaa cag gag ctt gag aaa   701

Asn Val Leu Val Lys Ala Lys Asn Ser Glu Glu Gln Glu Leu Glu Lys
                175                 180                 185

atg caa gaa ctt cag aag gaa atg cta gag aat ctc aag aaa aat gag   749

Met Gln Glu Leu Gln Lys Glu Met Leu Glu Asn Leu Lys Lys Asn Glu
            190                 195                 200

cat tcc atg aaa gtt gcc ata act gga gca ggt caa cca gtg aag acc   797

His Ser Met Lys Val Ala Ile Thr Gly Ala Gly Gln Pro Val Lys Thr
        205                 210                 215

ttc att cca gtt aca aaa cca gtg gat ttt cac ttt aaa acg gac gac   845

Phe Ile Pro Val Thr Lys Pro Val Asp Phe His Phe Lys Thr Asp Asp
    220                 225                 230

cgt ctc aag cgc act gcc aat cag cca gag ggg gat ggc tat aaa gcg   893

Arg Leu Lys Arg Thr Ala Asn Gln Pro Glu Gly Asp Gly Tyr Lys Ala
235                 240                 245                 250

gtg gac ttt gct tcg gag cta aga aaa cac cca cca tca cca gtt caa   941

Val Asp Phe Ala Ser Glu Leu Arg Lys His Pro Pro Ser Pro Val Gln
                255                 260                 265

gtt acc aaa gga ggg cac act gtt ccg aaa ccc ttc aac ctg tcc aag   989

Val Thr Lys Gly Gly His Thr Val Pro Lys Pro Phe Asn Leu Ser Lys
            270                 275                 280

ggc aaa cgt aag cat gag gag gct tca gat tac gtc tcc act gct gag   1037

Gly Lys Arg Lys His Glu Glu Ala Ser Asp Tyr Val Ser Thr Ala Glu
        285                 290                 295

cag gtt att gcc ttc tac aaa aga act cca gca cgt tat cac ctg cgc   1085

Gln Val Ile Ala Phe Tyr Lys Arg Thr Pro Ala Arg Tyr His Leu Arg
    300                 305                 310

agc cgc cag agg gag atg gag gga ccc tcc cca gtg aag atg atc aaa   1133

Ser Arg Gln Arg Glu Met Glu Gly Pro Ser Pro Val Lys Met Ile Lys
315                 320                 325                 330

aca aaa ctg acc aac cca aag acc cca ctg ctc caa acc aaa ggg cgt    1181

Thr Lys Leu Thr Asn Pro Lys Thr Pro Leu Leu Gln Thr Lys Gly Arg
                335                 340                 345

cat cgg cca gtc acg tgt aaa agt gct gca gag ctg gaa gct gag gaa    1229

His Arg Pro Val Thr Cys Lys Ser Ala Ala Glu Leu Glu Ala Glu Glu
            350                 355                 360

ctg gag atg ata aat cag tac aag ttt aag gct cag gaa ctg gac act    1277

Leu Glu Met Ile Asn Gln Tyr Lys Phe Lys Ala Gln Glu Leu Asp Thr
        365                 370                 375

aga atc ctg gaa ggg ggt cca gtc ctc ctt aag aag ccc ctt gtt aag    1325

Arg Ile Leu Glu Gly Gly Pro Val Leu Leu Lys Lys Pro Leu Val Lys
    380                 385                 390

gaa ccc act aaa gcc att ggt ttt gac ttg gaa ata gag aag aga atc    1373

Glu Pro Thr Lys Ala Ile Gly Phe Asp Leu Glu Ile Glu Lys Arg Ile
395                 400                 405                 410

caa cag cgg gag aag aaa gaa gaa att gaa gaa gag act ttc act ttc    1421

Gln Gln Arg Glu Lys Lys Glu Glu Ile Glu Glu Glu Thr Phe Thr Phe
                415                 420                 425

cac tct aga cct tgc cct tcc aaa atg ctg acc gat gtg gtg ggt gtc    1469

His Ser Arg Pro Cys Pro Ser Lys Met Leu Thr Asp Val Val Gly Val
            430                 435                 440

ccg ctg aag aag ctg ctc cca gtg aca gtg cct cag tct cct gct ttt    1517

Pro Leu Lys Lys Leu Leu Pro Val Thr Val Pro Gln Ser Pro Ala Phe
        445                 450                 455

gct ctg aag aac aga gta cgc att ccg gcc cag gaa gag aag gaa gag    1565

Ala Leu Lys Asn Arg Val Arg Ile Pro Ala Gln Glu Glu Lys Glu Glu
    460                 465                 470

atg gtg cca gtt atc aaa gcc act cgt atg cca cac tat ggg gtc cca 1613

Met Val Pro Val Ile Lys Ala Thr Arg Met Pro His Tyr Gly Val Pro
475                 480                 485                 490

ttc aag ccc aag ctc gta gaa cag cga caa gtg gac gtt tgt ccc ttt 1661

Phe Lys Pro Lys Leu Val Glu Gln Arg Gln Val Asp Val Cys Pro Phe
                495                 500                 505

tcc ttt tgt gac aga gac aag gag cga caa ctg cag aaa gag aag cga 1709

Ser Phe Cys Asp Arg Asp Lys Glu Arg Gln Leu Gln Lys Glu Lys Arg
            510                 515                 520

ttg gat gaa ctg cgc aaa gat gag gtc cct aaa ttc aag gct cag ccg 1757

Leu Asp Glu Leu Arg Lys Asp Glu Val Pro Lys Phe Lys Ala Gln Pro
        525                 530                 535

cta cca cag ttc gat aac atc cgt ctt cct gaa aag aag gtg aag atg 1805

Leu Pro Gln Phe Asp Asn Ile Arg Leu Pro Glu Lys Lys Val Lys Met
    540                 545                 550

ccg acc cag cag gag cca ttt gac ctc gag att gag aaa cgc gga gcc 1853

Pro Thr Gln Gln Glu Pro Phe Asp Leu Glu Ile Glu Lys Arg Gly Ala
555                 560                 565                 570

tcc aaa ttg cag cgg tgg cag cag cag atc caa gag gag ctg aag cag 1901

Ser Lys Leu Gln Arg Trp Gln Gln Gln Ile Gln Glu Glu Leu Lys Gln
                575                 580                 585

caa aaa gaa atg gtt gtg ttc aag gca cgg ccc aac act gtt gtc cac 1949

Gln Lys Glu Met Val Val Phe Lys Ala Arg Pro Asn Thr Val Val His
            590                 595                 600

caa gaa ccc ttt gtt ccc aag aag gaa aat agg agt ctt aca gag agc 1997

Gln Glu Pro Phe Val Pro Lys Lys Glu Asn Arg Ser Leu Thr Glu Ser
        605                 610                 615

ctt tct ggt tcc ata gtt caa gaa ggc ttt gag ctg gct aca gca aaa    2045

Leu Ser Gly Ser Ile Val Gln Glu Gly Phe Glu Leu Ala Thr Ala Lys
    620                 625                 630

cgg gcc aaa gag cgc cag gag ttt gac aag tgc ttg gca gag acg gaa    2093

Arg Ala Lys Glu Arg Gln Glu Phe Asp Lys Cys Leu Ala Glu Thr Glu
635                 640                 645                 650

gct cag aag agc ctt ttg gaa gag gag att cga aag cga cgg gaa gag    2141

Ala Gln Lys Ser Leu Leu Glu Glu Glu Ile Arg Lys Arg Arg Glu Glu
                655                 660                 665

gag gaa aag gaa gag atc agt cag ctg agg caa gag ctg gtg cac aag    2189

Glu Glu Lys Glu Glu Ile Ser Gln Leu Arg Gln Glu Leu Val His Lys
            670                 675                 680

gcc aag cct atc agg aag tac aga gct gtg gaa gtt aaa gcc agt gac    2237

Ala Lys Pro Ile Arg Lys Tyr Arg Ala Val Glu Val Lys Ala Ser Asp
        685                 690                 695

gtc cca ctt acc gtc ccc aga tcc ccc aac ttc tcg gac agg ttt aag    2285

Val Pro Leu Thr Val Pro Arg Ser Pro Asn Phe Ser Asp Arg Phe Lys
    700                 705                 710

tgt tga ttcgttttcc tgtgtcacag ccaaagccag ttttctgggt gtggttgcct    2341

Cys
715

gttcatgccc tggaccatat agtctgttga acaaaactgt gtccttttaa atagtggagt 2401

tgacgcaggg gcaagtgtct gctcattggg gttttgtaaa tactagatat taatggcctg 2461

gaggggcctg tttttaggtc ttccatgtga atacgttatg cttttattat gccctgtaat 2521

aaactgtgta aatgtaaagt ttgtgccgaa ttcgggaaat attcggacgt ttgtatgatg 2581

atgttgcact ctgtgacact gcgattttat tggctgtcat gtgtaacttc cttttccctt 2641

tcatgatttg cttgcaagta atggcaatta cagatggtca aatcctgcct gtaatttatc 2701

agcgtaaaag gagcagcaaa caccatttca agagttgaat acagacctgc ttatattaca 2761

aaattaataa cttctatgta tagttgtgaa acagatgcat aaatgactgt tcctggatcc 2821

tatagcaaca tttctgtaag ccacaccagc tcactcaccc agttcagcct gtctggaatt 2881

catttacagg agaatgtggc taagatgcca tattttatat actgaactta ttgcaccagt 2941

ctaaagtttc agcttcttca aatttgtcac gaggggtcac catctttgag ggagtctgcg 3001

acactcacat gctccgtgtg ctttgagaag ctgttgagaa gctaagattg ggggtcatca 3061

caaattttca agcagaaaat gaggttggcc tgtattataa gctgatgcta caaggctaat 3121

tcttaaattc tgatgctgat tgcactggtt tctgtgctgc tatgtagtat ctgtattggt 3181

tactaattag ccttatattg tgacattaag attttatgtt tactgtatat ttagtctatc 3241

cctggaccca gtgggtggca gcagcacaga gcatgtgcag tgagtaagcg gaggagagga 3301

tggggagcta ctggggcatc tttgagggca cggatcttta ctgttgaagg gttgtggttg 3361

ccttggctgg tacagtaaac tcaaaacata atgtacaaga tttctggccy wcttcgttgg 3421

ttagacttta gttctccttt atgccttgat gaagataccg attttcagta gctgtgctct 3481

ataattggtt tgaaaaccta ttaaactaag tccaccaaca aaaaaaaaaa aaaaaacc 3539

<210> 2
<211> 715
<212> PRT
<213> Xenopus laevis

<400> 2

Met Glu Asp Thr Gln Asp Thr Tyr Ser Tyr
1               5                   10

Asp Ala Pro Ser Ile Phe Asn Phe Ser Ser Phe His Glu Asp His Asn
                15                  20                  25

Ala Asp Ser Trp Phe Asp Gln Val Thr Asn Ala Glu Asn Ile
            30                  35                  40

Pro Pro Asp Gln Arg Arg Leu Ser Glu Thr Ser Val Asn Thr Glu Gln
                45                  50                  55

Asn Ser Lys Val Gln Pro Val Gln Thr Thr Pro Ser Lys Asp Asp Val
            60                  65                  70

Ser Asn Ser Ala Thr His Val Cys Asp Val Lys Ser Gln Ser Lys Arg
        75                  80                  85

Ser Ser Arg Arg Met Ser Lys Lys His Arg Gln Lys Leu Leu Val Lys
    90                  95                  100

Met Lys Asp Thr His Leu Glu Lys Glu Thr Ala Pro Pro Glu Tyr Pro
105                 110                 115                 120

Pro Cys Lys Lys Leu Lys Gly Ser Ser Ser Lys Gly Arg His Ala Pro
                125                 130                 135

Val Ile Lys Ser Gln Ser Thr Ser Ser His His Ser Met Thr Ser Pro
            140                 145                 150

Lys Pro Lys Ala Gln Leu Thr Met Pro Ser Thr Pro Thr Val Leu Lys
        155                 160                 165

Arg Arg Asn Val Leu Val Lys Ala Lys Asn Ser Glu Glu Gln Glu Leu
    170                 175                 180

Glu Lys Met Gln Glu Leu Gln Lys Glu Met Leu Glu Asn Leu Lys Lys
185                 190                 195                 200

Asn Glu His Ser Met Lys Val Ala Ile Thr Gly Ala Gly Gln Pro Val
                205                 210                 215

Lys Thr Phe Ile Pro Val Thr Lys Pro Val Asp Phe His Phe Lys Thr
            220                 225                 230

Asp Asp Arg Leu Lys Arg Thr Ala Asn Gln Pro Glu Gly Asp Gly Tyr
        235                 240                 245

Lys Ala Val Asp Phe Ala Ser Glu Leu Arg Lys His Pro Pro Ser Pro
    250                 255                 260

Val Gln Val Thr Lys Gly Gly His Thr Val Pro Lys Pro Phe Asn Leu
265                 270                 275                 280

Ser Lys Gly Lys Arg Lys His Glu Glu Ala Ser Asp Tyr Val Ser Thr
                285                 290                 295

Ala Glu Gln Val Ile Ala Phe Tyr Lys Arg Thr Pro Ala Arg Tyr His
            300                 305                 310

Leu Arg Ser Arg Gln Arg Glu Met Glu Gly Pro Ser Pro Val Lys Met
        315                 320                 325

Ile Lys Thr Lys Leu Thr Asn Pro Lys Thr Pro Leu Leu Gln Thr Lys
    330                 335                 340

Gly Arg His Arg Pro Val Thr Cys Lys Ser Ala Ala Glu Leu Glu Ala
345                 350                 355                 360

Glu Glu Leu Glu Met Ile Asn Gln Tyr Lys Phe Lys Ala Gln Glu Leu
                365                 370                 375

Asp Thr Arg Ile Leu Glu Gly Gly Pro Val Leu Leu Lys Lys Pro Leu
            380                 385                 390

Val Lys Glu Pro Thr Lys Ala Ile Gly Phe Asp Leu Glu Ile Glu Lys
        395                 400                 405

Arg Ile Gln Gln Arg Glu Lys Lys Glu Glu Ile Glu Glu Glu Thr Phe
    410                 415                 420

Thr Phe His Ser Arg Pro Cys Pro Ser Lys Met Leu Thr Asp Val Val
425                 430                 435                 440

Gly Val Pro Leu Lys Lys Leu Leu Pro Val Thr Val Pro Gln Ser Pro
                445                 450                 455

Ala Phe Ala Leu Lys Asn Arg Val Arg Ile Pro Ala Gln Glu Glu Lys
            460                 465                 470

Glu Glu Met Val Pro Val Ile Lys Ala Thr Arg Met Pro His Tyr Gly
        475                 480                 485

Val Pro Phe Lys Pro Lys Leu Val Glu Gln Arg Gln Val Asp Val Cys
    490                 495                 500

Pro Phe Ser Phe Cys Asp Arg Asp Lys Glu Arg Gln Leu Gln Lys Glu
505                 510                 515                 520

Lys Arg Leu Asp Glu Leu Arg Lys Asp Glu Val Pro Lys Phe Lys Ala
                525                 530                 535

Gln Pro Leu Pro Gln Phe Asp Asn Ile Arg Leu Pro Glu Lys Lys Val
            540                 545                 550

Lys Met Pro Thr Gln Gln Glu Pro Phe Asp Leu Glu Ile Glu Lys Arg
        555                 560                 565

Gly Ala Ser Lys Leu Gln Arg Trp Gln Gln Gln Ile Gln Glu Glu Leu
    570                 575                 580

Lys Gln Gln Lys Glu Met Val Val Phe Lys Ala Arg Pro Asn Thr Val
585                 590                 595                 600

Val His Gln Glu Pro Phe Val Pro Lys Lys Glu Asn Arg Ser Leu Thr
                605                 610                 615

Glu Ser Leu Ser Gly Ser Ile Val Gln Glu Gly Phe Glu Leu Ala Thr
            620                 625                 630

Ala Lys Arg Ala Lys Glu Arg Gln Glu Phe Asp Lys Cys Leu Ala Glu
        635                 640                 645

Thr Glu Ala Gln Lys Ser Leu Leu Glu Glu Glu Ile Arg Lys Arg Arg
    650                 655                 660

Glu Glu Glu Glu Lys Glu Glu Ile Ser Gln Leu Arg Gln Glu Leu Val
665                 670                 675                 680

His Lys Ala Lys Pro Ile Arg Lys Tyr Arg Ala Val Glu Val Lys Ala
                685                 690                 695

Ser Asp Val Pro Leu Thr Val Pro Arg Ser Pro Asn Phe Ser Asp Arg
            700                 705                 710

Phe Lys Cys
        715

<210> 3
<211> 3539
<212> DNA
<213> Xenopus laevis

<400> 3
ggcgggtttt tttttttaag actgattttg ggttgagatt acgcttcgta aattgggccg 60

tgcagaggaa ctagttggat ccagaagccc ttccacatac tgattcatag tgactgtagg 120

atattataga agcccgtgtc gccatggaag atacacagga cacctacagc tacgacgccc 180

cttctatttt caactttagc tcatttcatg aggatcacaa cgctgactcc tggttcgacc 240

aagtgaccaa tgcagaaaat attccccctg accagagacg gctctctgag acttctgtga 300

atactgagca aaattcaaag gtgcaaccag tacagaccac cccttcaaag gatgatgtct 360

ccaatagtgc tacacatgtt tgtgatgtga aatctcagtc aaagaggtca tccaggcgga 420

tgtctaagaa gcatcggcag aagcttctcg taaaaatgaa agacacacac ctggaaaaag 480

agactgcacc accggaatac ccaccgtgca aaaaattaaa ggggtccagt tctaaaggca 540

gacatgctcc agtaatcaag agccaatcca caagcagcca tcacagcatg acctctccaa 600

aaccgaaagc ccaactgacc atgccctcaa ctccaaccgt actgaagaga aggaatgtgc 660

ttgtaaaggc taaaaactca gaagaacagg agcttgagaa aatgcaagaa cttcagaagg 720

aaatgctaga gaatctcaag aaaaatgagc attccatgaa agttgccata actggagcag 780

gtcaaccagt gaagaccttc attccagtta caaaaccagt ggattttcac tttaaaacgg 840

acgaccgtct caagcgcact gccaatcagc cagaggggga tggctataaa gcggtggact 900

ttgcttcgga gctaagaaaa cacccaccat caccagttca agttaccaaa ggagggcaca 960

ctgttccgaa acccttcaac ctgtccaagg gcaaacgtaa gcatgaggag gcttcagatt 1020

acgtctccac tgctgagcag gttattgcct tctacaaaag aactccagca cgttatcacc 1080

tgcgcagccg ccagagggag atggagggac cctccccagt gaagatgatc aaaacaaaac 1140

tgaccaaccc aaagacccca ctgctccaaa ccaaagggcg tcatcggcca gtcacgtgta 1200

aaagtgctgc agagctggaa gctgaggaac tggagatgat aaatcagtac aagtttaagg 1260

ctcaggaact ggacactaga atcctggaag ggggtccagt cctccttaag aagccccttg 1320

ttaaggaacc cactaaagcc attggttttg acttggaaat agagaagaga atccaacagc 1380

gggagaagaa agaagaaatt gaagaagaga ctttcacttt ccactctaga ccttgccctt 1440

ccaaaatgct gaccgatgtg gtgggtgtcc cgctgaagaa gctgctccca gtgacagtgc 1500

ctcagtctcc tgcttttgct ctgaagaaca gagtacgcat tccggcccag gaagagaagg 1560

aagagatggt gccagttatc aaagccactc gtatgccaca ctatggggtc ccattcaagc 1620

ccaagctcgt agaacagcga caagtggacg tttgtccctt ttccttttgt gacagagaca 1680

aggagcgaca actgcagaaa gagaagcgat tggatgaact gcgcaaagat gaggtcccta 1740

aattcaaggc tcagccgcta ccacagttcg ataacatccg tcttcctgaa aagaaggtga 1800

agatgccgac ccagcaggag ccatttgacc tcgagattga gaaacgcgga gcctccaaat 1860

tgcagcggtg gcagcagcag atccaagagg agctgaagca gcaaaaagaa atggttgtgt 1920

tcaaggcacg gcccaacact gttgtccacc aagaaccctt tgttcccaag aaggaaaata 1980

ggagtcttac agagagcctt tctggttcca tagttcaaga aggctttgag ctggctacag 2040

caaaacgggc caaagagcgc caggagtttg acaagtgctt ggcagagacg gaagctcaga 2100

agagcctttt ggaagaggag attcgaaagc gacggaaaga ggaggaaaag gaagagatca 2160

gtcagctgag gcaagagctg gtgcacaagg ccaagcctat caggaagtac agagctgtgg 2220

aagttaaagc cagtgacgtc ccacttaccg tccccagatc ccccaacttc tcggacaggt 2280

ttaagtgttg attcgttttc ctgtgtcaca gccaaagcca gttttctggg tgtggttgcc 2340

tgttcatgcc ctggaccata tagtctgttg aacaaaactg tgtcctttta aatagtggag 2400

ttgacgcagg ggcaagtgtc tgctcattgg ggttttgtaa atactagata ttaatggcct 2460

ggaggggcct gtttttaggt cttccatgtg aatacgttat gcttttatta tgccctgtaa 2520

taaactgtgt aaatgtaaag tttgtgccga attcgggaaa tattcggacg tttgtatgat 2580

gatgttgcac tctgtgacac tgcgatttta ttggctgtca tgtgtaactt ccttttccct 2640

ttcatgattt gcttgcaagt aatggcaatt acagatggtc aaatcctgcc tgtaatttat 2700

cagcgtaaaa ggagcagcaa acaccatttc aagagttgaa tacagacctg cttatattac 2760

aaaattaata acttctatgt atagttgtga aacagatgca taaatgactg ttcctggatc 2820

ctatagcaac atttctgtaa gccacaccag ctcactcacc cagttcagcc tgtctggaat 2880

tcatttacag gagaatgtgg ctaagatgcc atattttata tactgaactt attgcaccag 2940

tctaaagttt cagcttcttc aaatttgtca cgaggggtca ccatctttga gggagtctgc 3000

gacactcaca tgctccgtgt gctttgagaa gctgttgaga agctaagatt gggggtcatc 3060

acaaattttc aagcagaaaa tgaggttggc ctgtattata agctgatgct acaaggctaa 3120

ttcttaaatt ctgatgctga ttgcactggt ttctgtgctg ctatgtagta tctgtattgg 3180

ttactaatta gccttatatt gtgacattaa gattttatgt ttactgtata tttagtctat 3240

ccctggaccc agtgggtggc agcagcacag agcatgtgca gtgagtaagc ggaggagagg 3300

atggggagct actggggcat ctttgagggc acggatcttt actgttgaag ggttgtggtt 3360

gccttggctg gtacagtaaa ctcaaaacat aatgtacaag atttctggcc ywcttcgttg 3420

gttagacttt agttctcctt tatgccttga tgaagatacc gattttcagt agctgtgctc 3480

tataattggt ttgaaaacct attaaactaa gtccaccaac aaaaaaaaaa aaaaaaacc 3539

<210> 4
<211> 2324
<212> DNA
<213> Homo sapiens

<400> 4
ctggagaaga gtatgaaaat gcagcaagag gtggtggaga tgcggaaaaa gaatgaagaa 60

ttcaagaaac ttgctctggc tggaataggg caacctgtga agaaatcagt gagccaggtc 120

accaaatcag ttgacttcca cttccgcaca gatgagcgaa tcaaacaaca tcctaagaac 180

caggaggaat ataaggaagt gaactttaca tctgaactac gaaagcatcc ttcatctcct 240

gcccgagtga ctaagggatg taccattgtt aagcctttca acctgtccca aggaaagaaa 300

agaacatttg atgaaacagt ttctacatat gtgccccttg cacagcagtt tgaagacttc 360

cataaacgaa cccctaacag atatcatttg aggagcaaga aggatgatat taacctgtta 420

ccctccaaat cttctgtgac caagatttgc agagacccac agactcctgt actgcaaacc 480

aaacaccgtg cacgggctgt gacctgcaaa agtacagcag agctgaaggc tgaggagctc 540

gagaaattgc aacaatacaa attcaaagca cgtgaacttg atcccagaat acttgaaggt 600

gggcccatct tgcccaagaa accacctgtg aaaccaccca ccgagcctat tggctttgat 660

ttggaaattg agaaaagaat ccaggagcga gaatcaaaga agaaaacaga ggatgaacac 720

tttgaatttc attccagacc ttgccctact aagattttgg aagatgttgt gggtgttcct 780

gaaaagaagg tacttccaat caccgtcccc aagtcaccag cctttgcatt gaagaacaga 840

attcgaatgc ccaccaaaga agatgaggaa gaggacgaac cggtagtgat aaaagctcaa 900

cctgtgccac attatggggt gccttttaag ccccaaatcc cagaggcaag aactgtggaa 960

atatgccctt tctcctttga ttctcgagac aaagaacgtc agttacagaa ggagaagaaa 1020

ataaaagaac tgcagaaagg ggaggtgccc aagttcaagg cacttccctt gcctcatttt 1080

gacaccatta acctgccaga gaagaaggta aagaatgtga cccagattga acctttctgc 1140

ttggagactg acagaagagg tgctctgaag gcacagactt ggaagcacca gctggaagaa 1200

gaactgagac agcagaaaga agcagcttgt ttcaaggctc gtccaaacac cgtcatctct 1260

caggagccct ttgttcccaa gaaagagaag aaatcagttg ctgagggcct ttctggttct 1320

ctagttcagg aaccttttca gctggctact gagaagagag ccaaagagcg gcaggagctg 1380

gagaagagaa tggctgaggt agaagcccag aaagcccagc agttggagga ggccagacta 1440

caggaggaag agcagaaaaa agaggagctg gccaggctac ggagagaact ggtgcataag 1500

gcaaatccaa tacgcaagta ccagggtctg gagataaagt caagtgacca gcctctgact 1560

gtgcctgtat ctcccaaatt ctccactcga ttccactgct aaactcagct gtgagctgcg 1620

gataccgccc ggcaatggga cctgctctta acctcaaacc taggaccgtc ttgctttgtc 1680

attgggcatg gagagaaccc atttctccag acttttacct acccgtgcct gagaaagcat 1740

acttgacaac tgtggactcc agttttgttg agaattgttt tcttacatta ctaaggctaa 1800

taatgagatg taactcatga atgtctcgat tagactccat gtagttactt cctttaaacc 1860

atcagccggc cttttatatg ggtcttcact ctgactagaa tttagtctct gtgtcagcac 1920

agtgtaatct ctattgctat tgccccttac gactctcacc ctctccccac tttttttaaa 1980

aattttaacc agaaaataaa gatagttaaa tcctaagata gagattaagt catggtttaa 2040

atgaggaaca atcagtaaat cagattctgt cctcttctct gcataccgtg aatttatagt 2100

taaggatccc tttgctgtga gggtagaaaa cctcaccaac tgcaccagtg aggaagaaga 2160

ctgcgtggat tcatggggag cctcacagca gccacgcagc aggctctggg tggggctgcc 2220

gttaaggcac gttctttcct tactggtgct gataacaaca gggaaccgtg cagtgtgcat 2280

tttaagacct ggcctggaat aaatacgttt tgtctttccc tccc 2324

<210> 5
<211> 533
<212> PRT
<213> Homo sapiens

<400> 5
Leu Glu Lys Ser Met Lys Met Gln Gln Glu Val Val Glu Met Arg Lys
1               5                   10                  15

Lys Asn Glu Glu Phe Lys Lys Leu Ala Leu Ala Gly Ile Gly Gln Pro
            20                  25                  30

Val Lys Lys Ser Val Ser Gln Val Thr Lys Ser Val Asp Phe His Phe
        35                  40                  45

Arg Thr Asp Glu Arg Ile Lys Gln His Pro Lys Asn Gln Glu Glu Tyr
    50                  55                  60

Lys Glu Val Asn Phe Thr Ser Glu Leu Arg Lys His Pro Ser Ser Pro
65                  70                  75                  80

Ala Arg Val Thr Lys Gly Cys Thr Ile Val Lys Pro Phe Asn Leu Ser
                85                  90                  95

Gln Gly Lys Lys Arg Thr Phe Asp Glu Thr Val Ser Thr Tyr Val Pro
            100                 105                 110

Leu Ala Gln Gln Val Glu Asp Phe His Lys Arg Thr Pro Asn Arg Tyr
        115                 120                 125

His Leu Arg Ser Lys Lys Asp Asp Ile Asn Leu Leu Pro Ser Lys Ser
    130                 135                 140

Ser Val Thr Lys Ile Cys Arg Asp Pro Gln Thr Pro Val Leu Gln Thr
145                 150                 155                 160

Lys His Arg Ala Arg Ala Val Thr Cys Lys Ser Thr Ala Glu Leu Lys
                165                 170                 175

Ala Glu Glu Leu Glu Lys Leu Gln Gln Tyr Lys Phe Lys Ala Arg Glu
            180                 185                 190

Leu Asp Pro Arg Ile Leu Glu Gly Gly Pro Ile Leu Pro Lys Lys Pro
        195                 200                 205

Pro Val Lys Pro Pro Thr Glu Pro Ile Gly Phe Asp Leu Glu Ile Glu
    210                 215                 220

Lys Arg Ile Gln Glu Arg Glu Ser Lys Lys Lys Thr Glu Asp Glu His
225                 230                 235                 240

Phe Glu Phe His Ser Arg Pro Cys Pro Thr Lys Ile Leu Glu Asp Val
                245                 250                 255

Val Gly Val Pro Glu Lys Lys Val Leu Pro Ile Thr Val Pro Lys Ser
            260                 265                 270

Pro Ala Phe Ala Leu Lys Asn Arg Ile Arg Met Pro Thr Lys Glu Asp
        275                 280                 285

Glu Glu Glu Asp Glu Pro Val Val Ile Lys Ala Gln Pro Val Pro His
    290                 295                 300

Tyr Gly Val Pro Phe Lys Pro Gln Ile Pro Glu Ala Arg Thr Val Glu
305                 310                 315                 320

Ile Cys Pro Phe Ser Phe Asp Ser Arg Asp Lys Glu Arg Gln Leu Gln
                325                 330                 335

Lys Glu Lys Lys Ile Lys Glu Leu Gln Lys Gly Glu Val Pro Lys Phe
            340                 345                 350

Lys Ala Leu Pro Leu Pro His Phe Asp Thr Ile Asn Leu Pro Glu Lys
        355                 360                 365

Lys Val Lys Asn Val Thr Gln Ile Glu Pro Phe Cys Leu Glu Thr Asp
    370                 375                 380

Arg Arg Gly Ala Leu Lys Ala Gln Thr Trp Lys His Gln Leu Glu Glu
385                 390                 395                 400

Glu Leu Arg Gln Gln Lys Glu Ala Ala Cys Phe Lys Ala Arg Pro Asn
                405                 410                 415

Thr Val Ile Ser Gln Glu Pro Phe Val Pro Lys Lys Glu Lys Lys Ser
            420                 425                 430

Val Ala Glu Gly Leu Ser Gly Ser Leu Val Gln Glu Pro Phe Gln Leu
        435                 440                 445

Ala Thr Glu Lys Arg Ala Lys Glu Arg Gln Glu Leu Glu Lys Arg Met
    450                 455                 460

Ala Glu Val Glu Ala Gln Lys Ala Gln Gln Leu Glu Glu Ala Arg Leu
465                 470                 475                 480

Gln Glu Glu Glu Gln Lys Lys Glu Glu Leu Ala Arg Leu Arg Arg Glu
                485                 490                 495

Leu Val His Lys Ala Asn Pro Ile Arg Lys Tyr Gln Gly Leu Glu Ile
            500                 505                 510

Lys Ser Ser Asp Gln Pro Leu Thr Val Pro Val Ser Pro Lys Phe Ser
        515                 520                 525

Thr Arg Phe His Cys
    530

<210> 6
<211> 2244
<212> DNA
<213> Homo sapiens

<220>
<221> CDS
<222> (1)..(2244)

<400> 6
atg tca caa gtt aaa agc tct tat tcc tat gat gcc ccc tcg gat ttc    48
atc aat ttt tca tcc ttg gat gat gaa gga gat act caa aac ata gat    96
tca tgg ttt gag gag aag gcc aat ttg gag aat aag tta ctg ggg aag    144
aat gga act gga ggg ctt ttt cag ggc aaa act cct ttg aga aag gct    192
aat ctt cag caa gct att gtc aca cct ttg aaa cca gtt gac aac act    240
tac tac aaa gag gca gaa aaa gaa aat ctt gtg gaa caa tcc att ccg    288
tca aat gct tgt tct tcc ctg gaa gtt gag gca gcc ata tca aga aaa     336
act cca gcc cag cct cag aga aga tct ctt agg ctt tct gct cag aag     384
gat ttg gaa cag aaa gaa aag cat cat gta aaa atg aaa gcc aag aga     432
tgt gcc act cct gta atc atc gat gaa att cta ccc tct aag aaa atg     480
aaa gtt tct aac aac aaa aag aag cca gag gaa gaa ggc agt gct cat     528
caa gat act gct gaa aac aat gca tct tcc cca gag aaa gcc aag ggt     576
aga cat act gtg cct tgt atg cca cct gca aag cag aag ttt cta aaa     624
agt act gag gag caa gag ctg gag aag agt atg aaa atg cag caa gag     672
gtg gtg gag atg cgg aaa aag aat gaa gaa ttc aag aaa ctt gct ctg     720
gct gga ata ggg caa cct gtg aag aaa tca gtg agc cag gtc acc aaa    768
tca gtt gac ttc cac ttc cgc aca gat gag cga atc aaa caa cat cct    816
aag aac cag gag gaa tat aag gaa gtg aac ttt aca tct gaa cta cga    864
aag cat cct tca tct cct gcc cga gtg act aag gga tgt acc att gtt    912
aag cct ttc aac ctg tcc caa gga aag aaa aga aca ttt gat gaa aca    960
gtt tct aca tat gtg ccc ctt gca cag caa gtt gaa gac ttc cat aaa    1008
cga acc cct aac aga tat cat ttg agg agc aag aag gat gat att aac    1056
ctg tta ccc tcc aaa tct tct gtg acc aag att tgc aga gac cca cag    1104
act cct gta ctg caa acc aaa cac cgt gca cgg gct gtg acc tgc aaa    1152
agt aca gca gag ctg gag gct gag gag ctc gag aaa ttg caa caa tac    1200
aaa ttc aaa gca cgt gaa ctt gat ccc aga ata ctt gaa ggt ggg ccc    1248
atc ttg ccc aag aaa cca cct gtg aaa cca ccc acc gag cct att ggc    1296
ttt gat ttg gaa att gag aaa aga atc cag gag cga gaa tca aag aag    1344
aaa aca gag gat gaa cac ttt gaa ttt cat tcc aga cct tgc cct act    1392
aag att ttg gaa gat gtt gtg ggt gtt cct gaa aag aag gta ctt cca    1440
atc acc gtc ccc aag tca cca gcc ttt gca ttg aag aac aga att cga    1488
atg ccc acc aaa gaa gat gag gaa gag gac gaa ccg gta gtg ata aaa    1536
gct caa cct gtg cca cat tat ggg gtg cct ttt aag ccc caa atc cca    1584
gag gca aga act gtg gaa ata tgc cct ttc tcg ttt gat tct cga gac    1632
aaa gaa cgt cag tta cag aag gag aag aaa ata aaa gaa ctg cag aaa    1680
ggg gag gtg ccc aag ttc aag gca ctt ccc ttg cct cat ttt gac acc    1728
att aac ctg cca gag aag aag gta aag aat gtg acc cag att gaa cct    1776
ttc tgc ttg gag act gac aga aga ggt gct ctg aag gca cag act tgg    1824
aag cac cag ctg gaa gaa gaa ctg aga cag cag aaa gaa gca gct tgt    1872
ttc aag gct cgt cca aac acc gtc atc tct cag gag ccc ttt gtt ccc    1920
aag aaa gag aag aaa tca gtt gct gag ggc ctt tct ggt tct cta gtt    1968
cag gaa cct ttt cag ctg gct act gag aag aga gcc aaa gag cgg cag    2016
gag ctg gag aag aga atg gct gag gta gaa gcc cag aaa gcc cag cag    2064
ttg gag gag gcc aga cta cag gag gaa gag cag aaa aaa gag gag ctg    2112
gcc agg cta cgg aga gaa ctg gtg cat aag gca aat cca ata cgc aag    2160
tac cag ggt ctg gag ata aag tca agt gac cag cct ctg act gtg cct    2208
gta tct ccc aaa ttc tcc act cga ttc cac tgc taa                    2244

<210> 7
<211> 748
<212> PRT
<213> Homo sapiens

<400> 7